(19) Europäisches Patentamt
European Patent Office
Office européen des brevets

(11) Veröffentlichungsnummer: **0 300 398**
**A2**

(12) **EUROPÄISCHE PATENTANMELDUNG**

(21) Anmeldenummer: **88111485.4**

(51) Int. Cl.⁴: **C07D 209/48 , A01N 37/32**

(22) Anmeldetag: **16.07.88**

(30) Priorität: **23.07.87 DE 3724395**

(43) Veröffentlichungstag der Anmeldung: **25.01.89 Patentblatt 89/04**

(84) Benannte Vertragsstaaten: **AT BE CH DE ES FR GB GR IT LI NL SE**

(71) Anmelder: **BASF Aktiengesellschaft**
**Carl-Bosch-Strasse 38**
**D-6700 Ludwigshafen(DE)**

(72) Erfinder: **Schwalge, Barbara, Dr.**
**Adolf-Diesterweg-Strasse 77**
**D-6700 Ludwigshafen(DE)**
Erfinder: **Plath, Peter, Dr.**
**Hans-Balcke-Strasse 13**
**D-6710 Frankenthal(DE)**
Erfinder: **Eicken, Karl, Dr.**
**Am Huettenwingert 12**
**D-6706 Wachenheim(DE)**
Erfinder: **Meyer, Norbert, Dr.**
**Dossenheimer Weg 22**
**D-6802 Ladenburg(DE)**
Erfinder: **Wuerzer, Bruno, Dr.**
**Ruedigerstrasse 13**
**D-6701 Otterstadt(DE)**

(54) **3-Phenylpropionsäurederivate.**

(57) 3-Phenylpropionsäurederivate der allgemeinen Formel I,

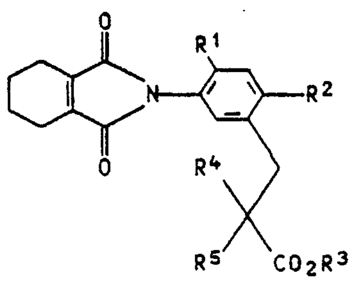

I

in der bedeutete
$R^1$ Wasserstoff oder Halogen,
$R^2$ Halogen,
$R^3$ unsubstituiertes oder durch Halogen, $C_1$-$C_4$-Alkoxy oder $C_1$-$C_4$-Alkylthio substituiertes $C_2$-$C_6$-Alkyl, unsubstituiertes oder durch $C_5$-$C_7$-Cycloalkyl substituiertes $C_1$-$C_6$-Alkyl, ferner $C_5$-$C_7$-Cycloalkyl, $C_3$-$C_6$-Alkenyl oder $C_3$-$C_6$-Alkinyl oder mit $R^4$ gemeinsam eine $C_2$-$C_4$-Alkylenkette,
$R^4$ Wasserstoff oder unsubstituiertes oder durch Halogen, $C_1$-$C_4$-Alkoxy oder $C_1$-$C_4$-Alkylthio substituiertes $C_2$-$C_6$-Alkyl, unsubstituiertes oder durch $C_5$-$C_7$-Cycloalkyl substituiertes $C_1$-$C_6$-Alkyl, $C_3$-$C_7$-Cycloalkyl, unsubstituiertes oder durch Halogen substituiertes $C_3$-$C_6$-Alkenyl oder $C_3$-$C_6$-Alkinyl oder Halogen oder in

EP 0 300 398 A2

der $R^4$ gemeinsam mit $R^5$ und dem Kohlenstoffatom dieses Substituenten $C_3$-$C_6$-Cycloalkyl bildet, das durch ein Sauerstoffatom oder ein Schwefelatom unterbrochen sein kann,
$R^5$ unsubstituiertes oder durch Halogen, $C_1$-$C_4$-Alkoxy oder $C_1$-$C_4$-Alkylthio substituiertes $C_1$-$C_6$-Alkyl, Cyano oder Acyl ($COR^6$) oder Alkoxycarbonyl ($CO_2R^7$), wobei bedeutet:
$R^6$ $C_1$-$C_6$ Alkyl oder gemeinsam mit $R^4$ eine $C_3$-$C_5$-Alkylenkette und
$R^7$ unsubstituiertes oder durch Halogen, $C_1$-$C_4$-Alkoxy oder $C_1$-$C_4$-Alkylthio substituiertes $C_2$-$C_6$-Alkyl oder unsubstituiertes oder durch $C_5$-$C_7$-Cycloalkyl substituiertes $C_1$-$C_6$-Alkyl, ferner $C_5$-$C_7$-Cycloalkyl, $C_3$-$C_6$-Alkenyl oder $C_3$-$C_6$-Alkinyl
mit der Maßgabe, daß $R^1$ und $R^4$ nicht gleichzeitig die Bedeutung Wasserstoff haben können, wenn $R^5$ Cyano oder $CO_2R^7$ ist,
Verfahren zu ihrer Herstellung und ihre Verwendung als Herbizide.

## 3-Phenylpropionsäurederivate

Es ist aus der DE-A-15 42 777 bekannt, daß substituierte Phenylpropionsäuren herbizid wirksam sind. Ähnliche Verbindungen, z.B. die nachstehend wiedergegebene Verbindung A, sind in EP-A-68 822 beschrieben

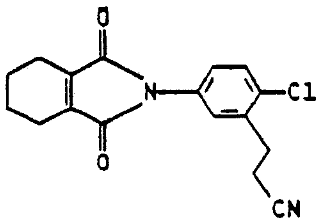

(A).

Besonders für die Anwendung neben Kulturpflanzen, z.B. im Nachauflaufverfahren sind jedoch Verbindungen wünschenswert, die bei geringerer Aufwandmenge höhere Selektivität aufweisen. Es wurde gefunden, daß substituierte Phenylpropionsäureverbindungen der allgemeinen Formel I

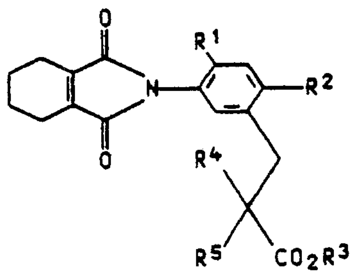

I

in der bedeutet:
$R^1$ Wasserstoff oder Halogen,
$R^2$ Halogen,
$R^3$ unsubstituiertes oder durch Halogen, $C_1$-$C_4$ Alkoxy oder $C_1$-$C_4$-Alkylthio substituiertes $C_2$-$C_6$-Alkyl, durch $C_5$-$C_7$-Cycloalkyl substituiertes $C_1$-$C_6$-Alkyl, ferner $C_5$-$C_7$-Cycloalkyl, $C_3$-$C_6$-Alkenyl oder $C_3$-$C_6$-Alkinyl oder mit $R^4$ gemeinsam eine $C_2$-$C_4$-Alkylenkette,
$R^4$ Wasserstoff oder unsubstituiertes oder durch Halogen, $C_1$-$C_4$-Alkoxy oder $C_1$-$C_4$-Alkylthio oder $C_5$-$C_7$-Cycloalkyl substituiertes $C_1$-$C_6$-Alkyl; $C_3$-$C_7$-Cycloalkyl; unsubstituiertes oder durch Halogen substituiertes $C_3$-$C_6$-Alkenyl oder $C_3$-$C_6$-Alkinyl oder Halogen oder
$R^4$ gemeinsam mit $R^5$ und dem Kohlenstoffatom dieses Substituenten $C_3$-$C_6$-Cycloalkyl, das durch ein Sauerstoffatom oder ein Schwefelatom unterbrochen sein kann,
$R^5$ unsubstituiertes $C_1$-$C_6$-Alkyl oder durch Halogen, $C_1$-$C_4$-Alkoxy oder $C_1$-$C_4$-Alkylthio substituiertes $C_2$-$C_6$-Alkyl, Cyan oder Acyl ($COR^6$) oder Alkoxycarbonyl ($CO_2R^7$), wobei bedeutet:
$R^6$ $C_1$-$C_6$-Alkyl oder gemeinsam mit $R^4$ eine $C_3$-$C_5$-Alkylenkette und
$R^7$ unsubstituiertes oder durch Halogen, $C_1$-$C_4$-Alkoxy oder $C_1$-$C_4$-Alkylthio substituiertes $C_2$-$C_6$-Alkyl oder unsubstituiertes oder durch $C_5$-$C_7$-Cycloalkyl substituiertes $C_1$-$C_6$-Alkyl, ferner $C_5$-$C_7$-Cycloalkyl, $C_3$-$C_6$-Alkenyl oder $C_3$-$C_6$-Alkinyl,
mit der Maßgabe, daß $R^1$ und $R^4$ nicht gleichzeitig die Bedeutung Wasserstoff haben können, wenn $R^5$ Cyano oder $CO_2R^7$ ist,
eine vorteilhafte herbizide Wirkung, besonders im Nachauflaufverfahren, haben und gegenüber einer Reihe von Kulturpflanzen selektiv sind.

In der Formel I ist unter Halogen Fluor, Chlor oder Brom zu verstehen, wobei $R^1$ in der Bedeutung Halogen bevorzugt für Fluor und $R^2$ bevorzugt für Chlor oder Brom stehen.

Die Bezeichnung Alkyl (allein oder als Teil eines Substituenten) umfaßt verzweigte und geradkettige Reste. Beispiele sind Methyl, Ethyl, n-Propyl, Isopropyl, n-Butyl, sec. Butyl, Isobutyl sowie die höheren Homologen Pentyl, Hexyl etc. samt ihren Isomeren. Sinngemäß enthalten Alkanoyle und Cyanalkyle ein zusätzliches Kohlenstoffatom.

Die Alkenyl- und Alkinylreste können ebenfalls verzweigt oder geradkettig sein. Beispiele für Alkenylreste in Formel I sind Allyl, 2-Propenyl, 2-Butenyl, 3-Butenyl, 2-Isobutenyl, 2-Pentenyl, 3-Pentenyl und 4-Pentenyl, insbesondere Allyl oder 2-Butenyl. Alkinylreste sind in der Regel Propargyl, 2-Butinyl, 3-Butinyl sowie isomere Pentinylreste; vorzugsweise ist der Alkinylrest jedoch ein Propargyl- oder 2- oder 3-Butinylrest.

Als Substituent der allgemeinen Formel I bedeutet Alkoxy: Methoxy, Ethoxy, n-Propyloxy, i-Propyloxy, die vier isomeren Butyloxyreste, insbesondere Methoxy, Ethoxy oder i-Propyloxy. Beispiele für Alkylthio in der Formel I sind Methylthio, Ethylthio, n-Propylthio, i-Propylthio oder n-Butylthio, insbesondere Methylthio und Ethylthio, Cycloalkylgruppen sind Cyclopropyl, Cyclobutyl, Cyclopentyl, Cyclohexyl und Cycloheptyl. Cycloaliphatische Reste entsprechen diesen Ringsystemen.

Mit "Alkylenkette" sind besonders die Propylen-, Butylen- und Pentylenkette gemeint. In ihnen kann - abgesehen von den entständigen Methylengruppen - jede durch ein Sauerstoff- oder Schwefelatom ersetzt sein. Vorzugsweise zu nennen sind Methylenoxamethylen-, Methylenoxaethylen-, Methylenoxapropylen -sowie Ethylenoxaethylenketten als Beispiele für Sauerstoff enthaltende Gruppierungen und Methylenthiamethylen-, Methylenthiaethylen-, Methylenthiapropylen- sowie Ethylenthiaethylenketten für die entsprechenden Schwefel enthaltenden Substituenten.

Die Phenylpropionsäurederivate der Formel I können als N-Tetrahydrophthalimide aufgefaßt werden; sie sind demnach aus 3.4.5.6-Tetrahydrophthalsäureanhydrid und einem Anilin-Derivat der Formel VI, z.B. in einem Lösungsmittel bei einer Temperatur von 20 bis 200 °C, vorzugsweise 40 bis 150 °C, erhältlich. Als Lösungsmittel eignen sich z.B. niedere Fettsäuren wie Eisessig oder Propionsäure oder aprotische Lösungsmittel. Beim Arbeiten in einem aprotischen Lösungsmittel verwendet man zweckmäßigerweise einen Wasserabscheider.

Unter den Verbindungen I sind diejenigen bevorzugt, in denen der Rest $R^1$ Wasserstoff oder Fluor und $R^2$ Chlor oder Brom bedeutet, wobei $R^1$ und $R^4$ nicht gleichzeitig die Bedeutung Wasserstoff haben können, wenn $R^5$ die Bedeutung Cyano oder Carbonsäurerest $CO_2R^7$ besitzt.

Die Anilin-Derivate der Formel VI können z.B. dadurch erhalten werden, daß man ein entsprechend substituiertes Carbonsäureesterderivat IV nitriert und das entstandene Nitrobenzol V anschließend mit Zinn-(II)ionen oder mit Eisen reduziert:

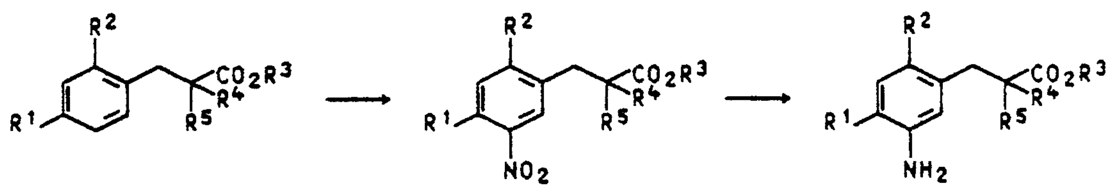

IV V VI

Die Reduktion kann auch durch katalytische Hydrierung mit einem Edelmetallkatalysator wie Platin oder Palladium bei relativ milden Bedingungen durchgeführt werden.

Die benötigten kernsubstituierten Phenylpropionsäureester der Formel IV sind auf verschiedene Weise herstellbar, z.B.:

a) wenn $R^5$ Cyano, $COR^6$ oder $CO_2R^7$ bedeutet:

indem man ein entsprechendes Benzylhalogenid II, vorzugsweise ein Chlorid oder Bromid, in an sich bekannter Weise in Gegenwart einer Base gegebenenfalls unter Katalysatorzusatz in einem Zweiphasensystem nach der in Chem. Ind. 18, 731 (1978) angegebenen Methode mit einem Carbonsäure-Derivat der Formel III umsetzt.

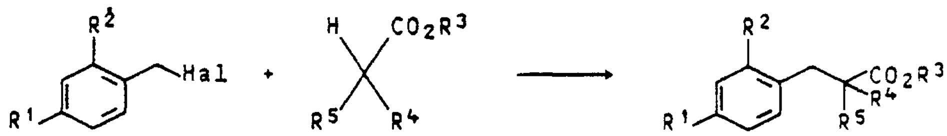

II III IV

Diese Umsetzung wird in einem aprotischen Lösungsmittel wie z.B. Toluol, Acetonitril oder Dimethylformamid unter Zweiphasenbedingungen mit einer anorganischen Base wie z.B. Natrium- oder Kaliumhydroxid in Gegenwart eines Phasentransferkatalysators vorgenommen; man verwendet vorzugsweise Kronenether wie 15-Krone-5 oder 18-Krone-6 bzw. entsprechende benzokondensierte Derivate wie z.B. Dibenzo-18-Krone-6.

In manchen Fällen können auch Polyethylenglycoldialkylether des Typs

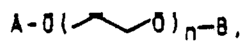

(n = 5-7 und A und B unabhängig voneinander $C_1$-$C_4$-Alkyl) oder quartäre Ammoniumsalze eingesetzt werden;

b) wenn $R^5$ Cyano, $COR^6$ oder $CO_2R^7$ bedeutet:

indem man ein, z.B. aus der zugrundeliegenden Zimtsäure erhältliches entsprechendes 3-Phenylpropionsäure-Derivat der Formel VII in Gegenwart einer Base in einem Lösungsmittel nach den in Organic Reactions 9, 107 (1957) angegebenen Methoden mit einem Halogenid der Formel VIII, vorzugsweise einem Iodid oder Bromid, umsetzt.

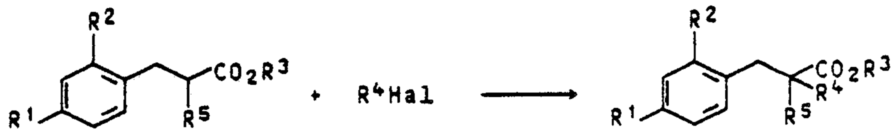


VII VIII IV

Verbindungen des Typs IV, in denen $R^4$ Halogen bedeutet, werden vorzugsweise in einem organischen Lösungsmittel in bekannter Weise durch Umsetzung mit einem Sulfurylhalogenid dargestellt.

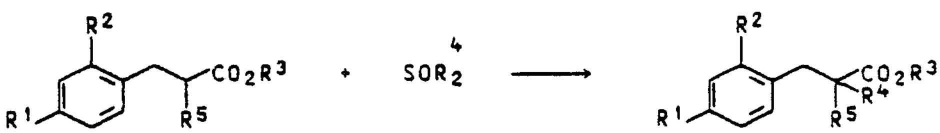


VII IV

Als Lösungsmittel eignen sich Carbonsäuren wie z. B. Eisessig oder Propionsäure oder Kohlenwasserstoffe wie z.B. Toluol. $R^4$ ist in diesem Fall vorzugsweise Chlor oder Brom.

Wahlweise kann in diesem Fall auch von einer Nitroverbindung XII

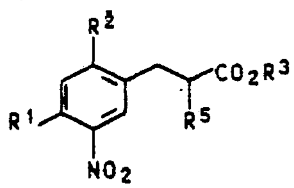

XII

ausgegangen werden, wobei man direkt zu Verbindungen des Typs V gelangt;

c) Für den Fall, daß $R^4$ nicht Wasserstoff oder Halogen und $R^5$ nicht Cyano, $COR^6$ oder $CO_2R^7$ bedeutet:

indem man ein entsprechendes Benzylhalogenid II, vorzugsweise ein Chlorid oder Bromid, in Gegenwart einer Base gegebenenfalls unter Katalysatorzusatz in einem aprotischen Lösungsmittel mit einem Aldehyd IX nach der in Lieb. Ann. Chem. 1979, 1585 angegebenen Methode zum Aldehyd X umsetzt und diesen zur Carbonsäure XI oxidiert. Veresterung liefert die gewünschten Verbindungen IV:

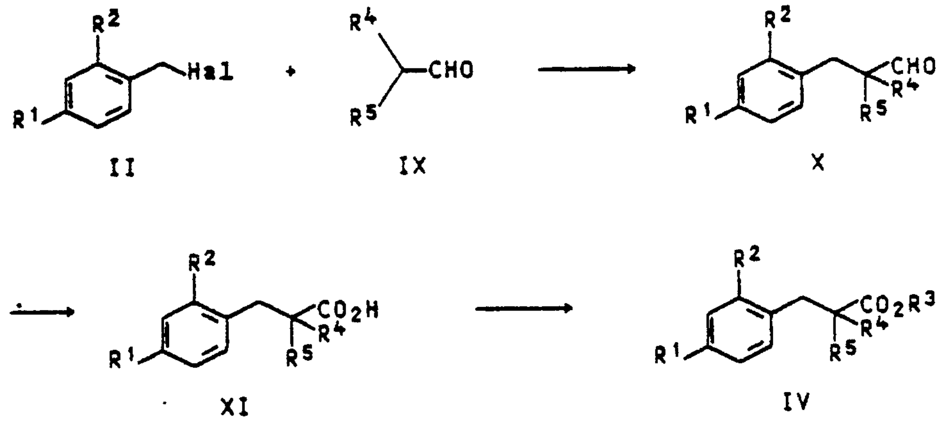

II IX X

XI IV

Die Umsetzung von II + IX zu X verläuft in einem Zweiphasensystem aus z.B. Toluol oder Acetonitril, und wäßrigem Alkalihydroxid in Gegenwart eines quartären Ammoniumsalzes wie Benzyltriethylammoniumchlorid, Tetrabutylammonium-iodid, · Tetrabutylammonium-hydroxid oder Tetrabutylammonium-hydrogensulfat.

Die in den nachstehenden Beispielen wiedergegebenen Arbeitsempfehlungen wurden unter entsprechender Abwandlung der Ausgangsverbindungen zur Gewinnung weiterer Verbindungen I benutzt; die erhaltenen Verbindungen sind mit physikalischen Angaben in den folgenden Tabellen aufgeführt; Verbindungen ohne solche Angaben lassen sich aus entsprechenden Stoffen in analoger Weise erhalten. Sie lassen aufgrund ihrer nahen strukturellen Beziehung zu den hergestellten und untersuchten Verbindungen eine gleichartige Wirkung erwarten.

Beispiel 1

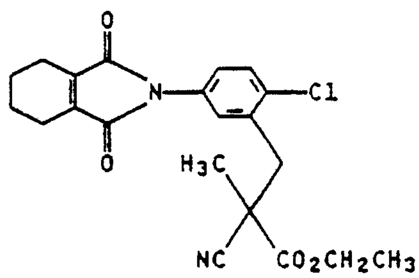

a) 64,4 g 2-Chlorbenzylchlorid, 76,0 g 2-Cyanpropionsäure-ethylester, 58,0 g trockenes Kaliumcarbonat und 0,1 g 18-Krone-6 werden unter Ausschluß von Feuchtigkeit 3 Stunden am Rückfluß erhitzt. Nach Abfiltrieren und Destillation des Rückstands erhält man 69,5 g 2-Chlorbenzyl-methylcyanessigsäure-ethylester ($Kp_{0,1}$: 107-109 °C).

b) Zu 25,2 g des Esters gibt man 41,7 ml konz. Salpetersäure (d = 1,51) bei 0 bis 5 °C in 30 Minuten und rührt weitere 30 Minuten. Man rührt in 400 ml Eiswasser ein und extrahiert zweimal mit je 70 ml Toluol. Nach Waschen mit 10 %iger Natriumbicarbonat-Lösung und Wasser, Verdampfen des Lösungsmittels und Umkristallisation aus 50 ml Diisopropylether gewinnt man 18,9 g (2-Chlor-5-nitrobenzyl)-methylcyanessigsäure-ethylester (Fp.: 72-74 °C).

c) Zu einer Mischung von 10,6 g Eisenpulver in 70 ml Methanol und 15 ml Eisessig tropft man bei Rückfluß in einer Stunde eine Lösung von 18,7 g der Nitroverbindung in 28 ml Methanol und 40 ml Eisessig. Nach weiteren 30 Minuten Sieden am Rückfluß wird abgesaugt und mit 50 ml Essigester nachgewaschen. Man rührt in 800 ml Wasser ein und extrahiert dreimal mit je 100 ml Essigester. Nach Trocknen und Verdampfen des Lösungsmittels i.V. isoliert man 15,5 g (2-Chlor-5-aminobenzyl)-methylcyanessigsäure-ethylester als Flüssigkeit (RT).

d) 15,5 g der Aminoverbindung und 9,3 g Cyclohexen-1,2-dicarbonsäureanhydrid werden in 150 ml Eisessig 5 Stunden bei 50 °C gerührt. Nach dem Verdampfen des Lösungsmittels i.V. isoliert man aus dem Rückstand (23,0 g) durch Umkristallisation aus 80 ml Methanol 14,4 g 2-Chlor-5-(N-3,4,5,6-tetrahydrophthal-

säureimido)-benzyl-methylcyanessigsäure-ethylester (Fp.: 111-113 °C; Wirkstoffbeispiel 2.020).

Weitere Beispiele für Wirkstoffe die nach diesem Syntheseprinzip hergestellt werden können finden sich in den Tabellen 2, 3 und 4.

Beispiel 2

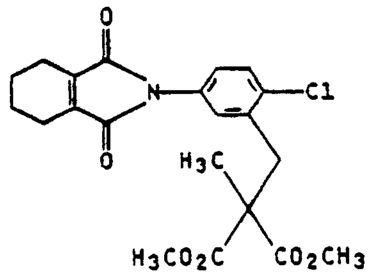

a) Man legt unter Feuchtigkeitsausschluß bei Raumtemperatur 3 g Natriumhydrid (80 %ige Suspension in Leinöl) in 150 ml Dimethylformamid vor und tropft 25,7 g 2-Chlorbenzylmalonsäure-dimethylester (J.Am.Chem. Soc. 71, 2644 (1949)) in 50 ml Dimethylformamid zu. Nach beendeter Wasserstoffentwicklung versetzt man mit 14,2 g Methyliodid in 50 ml Dimethylformamid und läßt 16 Stunden nachrühren. Man gießt auf 300 ml Eiswasser und kristallisiert in der Kälte. Nach Absaugen und Trocknen erhält man 23,5 g 2-Chlorbenzyl-methylmalonsäure-dimethylester (Fp.: 52-53 °C).

b) 23,5 g des Diesters werden mit 36,1 ml konz. Salpetersäure (D = 1,51) bei 0-5 °C nitriert. Aus der Toluolphase erhält man nach Waschen mit 10 %iger Natriumbicarbonat-Lösung und Wasser und Abziehen des Lösungsmittels i.V. 23,4 g (2-Chlor-5-nitrobenzyl)-methylmalonsäure-dimethylester als Flüssigkeit (RT).

c) 23,4 g der Nitroverbindung werden mit 11,1 g Eisenpulver in insgesamt 55 ml Eisessig und 100 ml Methanol reduziert. Aus dem Rohprodukt (20,8 g) erhält man durch Extrahieren mit Ethylacetat 16,7 g (2-Chlor-5-aminobenzyl)-methylmalonsäure-dimethylester als Flüssigkeit (RT).

d) Aus 16,7 g der Aminoverbindung und 10,2 g Cyclohexen-1,2-dicarbonsäureanhydrid in 250 ml Eisessig erhält man 26,8 g Rohprodukt, das nach Chromatographie an Kieselgel mit Toluol/1 % Aceton 13,7 g [2-Chlor-5-(N-3.4.5.6-tetrahydrophthalsäureamido)-benzyl]-methylmalonsäure-dimethylester (Fp.: 103-105 °C (Wirkstoffbeispiel 3.010) liefert.

Weitere Beispiele für Wirkstoffe, die nach diesem Syntheseprinzip hergestellt werden können, finden sich in Tabelle 3.

Beispiel 3

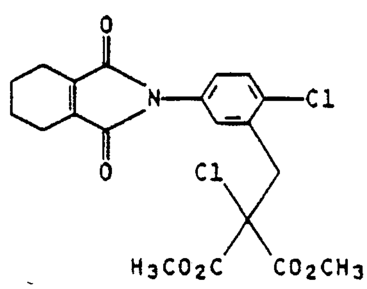

a) Zu einer Lösung von 51,3 g 2-Chlorbenzylmalonsäure-dimethylester (J.Am.Chem.Soc. 71, 2644 (1949)) in 80 ml konz. Schwefelsäure wird unter Rühren bei 3 bis 5 °C eine Mischung aus 9 ml konz. Salpetersäure (d = 1,51) und 20 ml konz. Schwefelsäure in 1 Stunde zugetropft und 2 Stunden bei gleicher Temperatur nachgerührt. Nach Einrühren in 1 l Eiswasser, Extraktion mit zweimal je 100 ml Toluol, Waschen mit 10 %iger Natriumbicarbonatlösung und Wasser isoliert man 58,3 g kristallines Rohprodukt, das nach Umkristallisieren aus 120 ml Diisopropylether 40,0 g (2-Chlor-5-nitrobenzyl)-malonsäure-dimethylester (Fp.: 72-74 °C) liefert.

b) Zu einer Lösung von 20,0 g der Nitroverbindung in 80 ml Eisessig tropft man bei 80 bis 85 °C 9,5 g Sulfurylchlorid und erhitzt 2 Stunden am Rückfluß. Nach dem Verdampfen des Lösungsmittels wird mit 150 ml Wasser verrührt, abgesaugt und mit 70 ml 5 %iger Natriumbicarbonat-Lösung angeteigt. Aus dem Rohprodukt (19,4 g, Fp: 69-74 °C)isoliert man durch Umkristallisation aus 50 ml Methanol 14,9 g (2-Chlor-5-nitrobenzyl)-chlormalonsäure-dimethylester (Fp: 75-78 °C).

c) 14,9 g der Nitroverbindung werden mit 7,5 g Eisenpulver in insgesamt 38 ml Eisessig und 70 ml Methanol reduziert. Aus dem Rohprodukt (13,8 g) erhält man durch Anteigen mit 50 ml Diisopropylether 10,2 g (2-Chlor-5-aminobenzyl)-chlormalonsäure-dimethylester (Fp: 107-109 °C).

d) Aus 10,0 g der Aminoverbindung und 5,2 g Cyclohexen-1,2-dicarbonsäureanhydrid in 130 ml Eisessig erhält man 15,0 g Rohprodukt, das nach Umkristallisation aus 200 ml Methanol 9,5 g [2-Chlor-5-(N-3,4,5,6-tetrahydrophthalsäureimido)-benzyl]-chlormalonsäure-dimethylester (Fp: 131-133 °C; Wirkstoffbeispiel 3.002) liefert.

Weitere Beispiele für Wirkstoffe, die nach diesem Syntheseprinzip hergestellt werden können, finden sich in Tabelle 2 und 3.

Beispiel 4

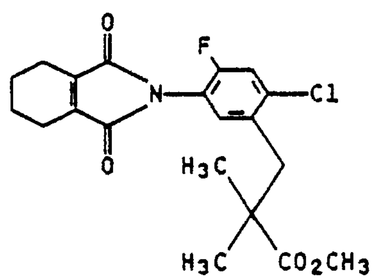

a) Zu einer Mischung aus 1,9 g Tetrabutylammonium-iodid, 155 g 30 %iger Natronlauge und 115 ml Toluol tropft man unter intensiver Vermischung bei 80 °C gleichzeitig, aber getrennt 65,0 g 2-Chlor-4-fluorbenzylchlorid in 90 ml Toluol und 28,8 g i-Butyraldehyd in 30 Minuten zu und rührt 3 Stunden bei 80 °C nach. Die organische Phase wird abgetrennt, dreimal mit je 50 ml Wasser gewaschen, getrocknet und über Kurzwegdestillation ($Kp_{0,3}$ = 70-90 °C) konzentriert (58,5 g). Destillation liefert 45,1 g (2-Chlor-4-fluorphenyl)-pivalaldehyd ($Kp_{0,1}$: 53-55 °C).

b) Zu einer Lösung von 90,0 g obigen Aldehyds in 200 ml Dioxan werden 54,5 g Amidosulfonsäure und eine Lösung von 100,1 g Dinatriumhydrogenphosphat in 200 ml Wasser gegeben, bei Raumtemperatur eine Lösung von 57,0 g 80 %igem Natriumchlorit in 200 ml Wasser in 45 Minuten unter Rühren bei pH = 4 zugetropft und 6 Stunden bei gleicher Temperatur nachgerührt. Nach dem Abziehen des Dioxans i.V. wird dreimal mit je 250 ml Ether extrahiert, einmal mit 100 ml Wasser und dreimal mit je 120 ml 2n Natronlauge extrahiert. Der Natronlauge-Extrakt wird einmal mit 100 ml Ether gewaschen und nach Ansäuern mit halbkonzentrierter Salzsäure auf pH = 3 dreimal mit je 250 ml Ether ausgeschüttelt. Man isoliert 74,8 g (2-Chlor-4-fluorphenyl)-pivalinsäure (Fp: 43-44 °C).

c) Aus 74,5 g der Säure in 140 ml konz. Schwefelsäure und 16,2 ml Salpetersäure (d = 1,51) in 30 ml konz. Schwefelsäure erhält man 86 g der rohen Nitroverbindung, die nach Umkristallisieren aus 450 ml Cyclohexan 70,8 g (2-Chlor-4-fluor-5-nitrophenyl)-pivalinsäure (Fp: 102-104 °C) liefert.

d) Zu 13,2 g der Nitroverbindung in 130 ml Toluol werden nach Zugabe von 1 Tropfen DMF bei 90-100 °C 5,3 ml Thionylchlorid getropft und bei 100-110 °C nachgerührt bis die Gasentwicklung beendet ist (ca. 1 Stunde). Nach dem Verdampfen des Lösungsmittels i.V. wird der Rückstand dreimal mit je 30 ml Toluol aufgenommen und erneut i.V. eingeengt. Der Rückstand (15,0 g) wird bei 10 °C in 50 ml Methanol aufgenommen und bei 5 °C tropfenweise mit 6,5 ml α-Picolin versetzt. Nach 24 Stunden Rühren wird zur Trockene gebracht und mit 150 ml Ether und 30 ml 2n Salzsäure aufgenommen. Aus der Etherphase isoliert man nach Waschen mit Wasser, Trocknen und Verdampfen des Ethers 12,8 g (2-Chlor-4-fluor-5-nitrophenyl)-pivalinsäure-methylester (Flüssigkeit bei RT).

e) Aus 12,7 g des nitrierten Esters und 16,7 g Eisenpulver in insgesamt 100 ml Methanol und 55 ml Eisessig erhält man 10,8 g (2-Chlor-4-fluor-5-aminophenyl)-pivalinsäure-methylester (Flüssigkeit bei RT).

f) Aus 10,6 g der Aminoverbindung und 6,5 g Cyclohexen-1,2-dicarbonsäureanhydrid in 80 ml Eisessig erhält man 16.0 g Rohprodukt, nach Chromatographie an 60 g Kieselgel mit Toluol 12,3 g [2-Chlor-4-fluor-5-(N-3.4.5.6-tetrahydrophthalimido)-phenyl]-pivalinsäure-methylester (Fp: 68-70 ° C; Wirkstoffbeispiel 1.020).

Weitere Beispiele für Wirkstoffe, die nach diesem Syntheseprinzip hergestellt werden können, finden sich in Tabelle 1.

Tabelle 1

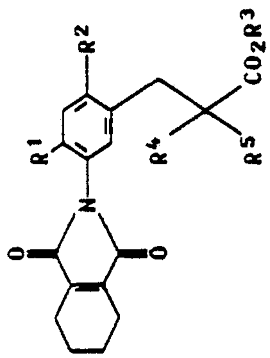

| Nr. | $R^1$ | $R^2$ | $R^3$ | $R^4$ | $R^5$ | Fp |
|---|---|---|---|---|---|---|
| 1.001 | H | Cl | $CH_3$ | H | $CH_3$ | |
| 1.002 | F | Cl | $CH_3$ | H | $CH_3$ | |
| 1.003 | H | Cl | $CH_2CH_3$ | H | $CH_3$ | |
| 1.004 | F | Cl | $CH_2CH_3$ | H | $CH_3$ | |
| 1.005 | H | Cl | $CH_2CH_2OCH_3$ | H | $CH_3$ | |
| 1.006 | F | Cl | $CH_2CH_2OCH_3$ | H | $CH_3$ | |
| 1.007 | H | Cl | $CH_3$ | H | $CH_2CH_3$ | |
| 1.008 | F | Cl | $CH_3$ | H | $CH_2CH_3$ | |
| 1.009 | H | Cl | $CH_2CH_3$ | H | $CH_2CH_3$ | |
| 1.010 | F | Cl | $CH_2CH_3$ | H | $CH_2CH_3$ | |
| 1.011 | H | Cl | $CH_2CH_2OCH_3$ | H | $CH_2CH_3$ | |
| 1.012 | F | Cl | $CH_2CH_2OCH_3$ | H | $CH_2CH_3$ | |
| 1.013 | H | Cl | $CH_3$ | H | $CH_2CH_2CH_3$ | |
| 1.014 | F | Cl | $CH_3$ | H | $CH_2CH_2CH_3$ | |
| 1.015 | H | Cl | $CH_2CH_3$ | H | $CH_2CH_2CH_3$ | |
| 1.016 | F | Cl | $CH_2CH_3$ | H | $CH_2CH_2CH_3$ | |
| 1.017 | H | Cl | $CH_2CH_2OCH_3$ | H | $CH_2CH_2CH_3$ | |
| 1.018 | F | Cl | $CH_2CH_2OCH_3$ | H | $CH_2CH_2CH_3$ | |

| Nr. | $R^1$ | $R^2$ | $R^3$ | $R^4$ | $R^5$ | Fp |
|---|---|---|---|---|---|---|
| 1.019 | H | Cl | $CH_3$ | $CH_3$ | $CH_3$ | 103- 4°C |
| 1.020 | F | Cl | $CH_3$ | $CH_3$ | $CH_3$ | 68-70°C |
| 1.021 | Cl | Cl | $CH_3$ | $CH_3$ | $CH_3$ | 122- 3°C |
| 1.022 | H | Cl | $CH_2CH_3$ | $CH_3$ | $CH_3$ | |
| 1.023 | F | Cl | $CH_2CH_3$ | $CH_3$ | $CH_3$ | Öl |
| 1.024 | Cl | Cl | $CH_2CH_3$ | $CH_3$ | $CH_3$ | 88-89°C |
| 1.025 | H | Cl | $CH_2CH_2CH_2CH_3$ | $CH_3$ | $CH_3$ | |
| 1.026 | F | Cl | $CH_2CH_2CH_2CH_3$ | $CH_3$ | $CH_3$ | |
| 1.027 | Cl | Cl | $CH_2CH_2CH_2CH_3$ | $CH_3$ | $CH_3$ | |
| 1.028 | H | Cl | $CH_2CH_2CH(CH_3)_2$ | $CH_3$ | $CH_3$ | |
| 1.029 | F | Cl | $CH_2CH_2CH(CH_3)_2$ | $CH_3$ | $CH_3$ | 98-99°C |
| 1.030 | Cl | Cl | $CH_2CH_2CH(CH_3)_2$ | $CH_3$ | $CH_3$ | 112°C |
| 1.031 | H | Cl | $CH_2CH_2OCH_3$ | $CH_3$ | $CH_3$ | Öl |
| 1.032 | F | Cl | $CH_2CH_2OCH_3$ | $CH_3$ | $CH_3$ | Öl |
| 1.033 | Cl | Cl | $CH_2CH_2OCH_3$ | $CH_3$ | $CH_3$ | Öl |
| 1.034 | H | Cl | $CH_2CH_2OCH_2CH_3$ | $CH_3$ | $CH_3$ | |
| 1.035 | F | Cl | $CH_2CH_2OCH_2CH_3$ | $CH_3$ | $CH_3$ | 63- 4°C |
| 1.036 | Cl | Cl | $CH_2CH_2OCH_2CH_3$ | $CH_3$ | $CH_3$ | Öl |
| 1.037 | H | Cl | $CH_2CH_2SCH_2CH_3$ | $CH_3$ | $CH_3$ | |
| 1.038 | F | Cl | $CH_2CH_2SCH_2CH_3$ | $CH_3$ | $CH_3$ | |
| 1.039 | H | Cl | 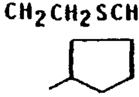 | $CH_3$ | $CH_3$ | |
| 1.040 | F | Cl | 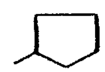 | $CH_3$ | $CH_3$ | |
| 1.041 | H | Cl | $CH_2CH=CH_2$ | $CH_3$ | $CH_3$ | |
| 1.042 | F | Cl | $CH_2CH=CH_2$ | $CH_3$ | $CH_3$ | |
| 1.043 | H | Cl | $CH_2C\equiv CH$ | $CH_3$ | $CH_3$ | |
| 1.044 | F | Cl | $CH_2C\equiv CH$ | $CH_3$ | $CH_3$ | |

| Nr. | $R^1$ | $R^2$ | $R^3$ | $R^4$ | $R^5$ | Fp |
|---|---|---|---|---|---|---|
| 1.045 | H | Cl | $CH_3$ | $CH_3$ | $CH_2CH_3$ | |
| 1.046 | F | Cl | $CH_3$ | $CH_3$ | $CH_2CH_3$ | Öl |
| 1.047 | H | Cl | $CH_2CH_3$ | $CH_3$ | $CH_2CH_3$ | |
| 1.048 | F | Cl | $CH_2CH_3$ | $CH_3$ | $CH_2CH_3$ | |
| 1.049 | H | Cl | $CH_2CH_2OCH_3$ | $CH_3$ | $CH_2CH_3$ | |
| 1.050 | F | Cl | $CH_2CH_2OCH_3$ | $CH_3$ | $CH_2CH_3$ | |
| 1.051 | H | Cl | $CH_3$ | $CH_3$ | $CH_2CH_2CH_3$ | |
| 1.052 | F | Cl | $CH_3$ | $CH_3$ | $CH_2CH_2CH_3$ | |
| 1.053 | H | Cl | $CH_2CH_3$ | $CH_3$ | $CH_2CH_2CH_3$ | |
| 1.054 | F | Cl | $CH_2CH_3$ | $CH_3$ | $CH_2CH_2CH_3$ | |
| 1.055 | H | Cl | $CH_2CH_2OCH_3$ | $CH_3$ | $CH_2CH_2CH_3$ | |
| 1.056 | F | Cl | $CH_2CH_2OCH_3$ | $CH_3$ | $CH_2CH_2CH_3$ | |
| 1.057 | H | Cl | $CH_3$ | $CH_2CH_3$ | $CH_2CH_3$ | 103-105°C |
| 1.058 | F | Cl | $CH_3$ | $CH_2CH_3$ | $CH_2CH_3$ | Öl |
| 1.059 | H | Cl | $CH_2CH_3$ | $CH_2CH_3$ | $CH_2CH_3$ | |
| 1.060 | F | Cl | $CH_2CH_3$ | $CH_2CH_3$ | $CH_2CH_3$ | |
| 1.061 | H | Cl | $CH_2CH_2OCH_3$ | $CH_2CH_3$ | $CH_2CH_3$ | |
| 1.062 | F | Cl | $CH_2CH_2OCH_3$ | $CH_2CH_3$ | $CH_2CH_3$ | |
| 1.063 | H | Cl | $CH_3$ | $CH_2CH_3$ | $CH_2CH_2CH_3$ | |
| 1.064 | F | Cl | $CH_3$ | $CH_2CH_3$ | $CH_2CH_2CH_3$ | |
| 1.065 | H | Cl | $CH_2CH_3$ | $CH_2CH_3$ | $CH_2CH_2CH_3$ | |
| 1.066 | F | Cl | $CH_2CH_3$ | $CH_2CH_3$ | $CH_2CH_2CH_3$ | |
| 1.067 | H | Cl | $CH_2CH_2OCH_3$ | $CH_2CH_3$ | $CH_2CH_2CH_3$ | |
| 1.068 | F | Cl | $CH_2CH_2OCH_3$ | $CH_2CH_3$ | $CH_2CH_2CH_3$ | |
| 1.069 | H | Cl | $CH_3$ | $CH_2CH_2CH_3$ | $CH_2CH_2CH_3$ | |
| 1.070 | F | Cl | $CH_3$ | $CH_2CH_2CH_3$ | $CH_2CH_2CH_3$ | |

| Nr. | $R^1$ | $R^2$ | $R^3$ | $R^4$ | $R^5$ | Fp |
|---|---|---|---|---|---|---|
| 1.071 | H | Cl | $CH_2CH_3$ | $CH_2CH_2CH_3$ | $CH_2CH_2CH_3$ | |
| 1.072 | F | Cl | $CH_2CH_3$ | $CH_2CH_2CH_3$ | $CH_2CH_2CH_3$ | |
| 1.073 | H | Cl | $CH_2CH_2OCH_3$ | $CH_2CH_2CH_3$ | $CH_2CH_2CH_3$ | |
| 1.074 | F | Cl | $CH_2CH_2OCH_3$ | $CH_2CH_2CH_3$ | $CH_2CH_2CH_3$ | |
| 1.075 | H | Cl | $CH_3$ | $CH_2CH_2CH_2CH_2$ | | 108-109°C |
| 1.076 | F | Cl | $CH_3$ | $CH_2CH_2CH_2CH_2$ | | 65-70°C |
| 1.077 | H | Cl | $CH_2CH_3$ | $CH_2CH_2CH_2CH_2$ | | |
| 1.078 | F | Cl | $CH_2CH_3$ | $CH_2CH_2CH_2CH_2$ | | |
| 1.079 | H | Cl | $CH_2CH_2OCH_3$ | $CH_2CH_2CH_2CH_2$ | | |
| 1.080 | F | Cl | $CH_2CH_2OCH_3$ | $CH_2CH_2CH_2CH_2$ | | |
| 1.081 | H | Cl | $CH_3$ | $CH_2CH_2CH_2CH_2CH_2$ | | |
| 1.082 | F | Cl | $CH_3$ | $CH_2CH_2CH_2CH_2CH_2$ | | 131-4°C |
| 1.083 | H | Cl | $CH_2CH_3$ | $CH_2CH_2CH_2CH_2CH_2$ | | |
| 1.084 | F | Cl | $CH_2CH_3$ | $CH_2CH_2CH_2CH_2CH_2$ | | |
| 1.085 | H | Cl | $CH_2CH_2OCH_3$ | $CH_2CH_2CH_2CH_2CH_2$ | | |
| 1.086 | F | Cl | $CH_2CH_2OCH_3$ | $CH_2CH_2CH_2CH_2CH_2$ | | |
| 1.087 | H | Cl | $CH_3$ | $CH_2CH_2OCH_2$ | | |
| 1.088 | F | Cl | $CH_3$ | $CH_2CH_2OCH_2$ | | |
| 1.089 | H | Cl | $CH_2CH_3$ | $CH_2CH_2OCH_2$ | | |
| 1.090 | F | Cl | $CH_2CH_3$ | $CH_2CH_2OCH_2$ | | |
| 1.091 | H | Cl | $CH_2CH_2OCH_3$ | $CH_2CH_2OCH_2$ | | |
| 1.092 | F | Cl | $CH_2CH_2OCH_3$ | $CH_2CH_2OCH_2$ | | |
| 1.093 | H | Cl | $CH_3$ | $CH_2CH_2CH_2OCH_2$ | | |
| 1.094 | F | Cl | $CH_3$ | $CH_2CH_2CH_2OCH_2$ | | |
| 1.095 | H | Cl | $CH_2CH_3$ | $CH_2CH_2CH_2OCH_2$ | | |
| 1.096 | F | Cl | $CH_2CH_3$ | $CH_2CH_2CH_2OCH_2$ | | |

| Nr. | $R^1$ | $R^2$ | $R^3$ | $R^4$ $R^5$ | Fp |
|---|---|---|---|---|---|
| 1.097 | H | Cl | $CH_2CH_2OCH_3$ | $CH_2CH_2CH_2OCH_2$ | |
| 1.098 | F | Cl | $CH_2CH_2OCH_3$ | $CH_2CH_2CH_2OCH_2$ | |
| 1.099 | H | Cl | $CH_3$ | $CH_2CH_2OCH_2CH_2$ | |
| 1.100 | F | Cl | $CH_3$ | $CH_2CH_2OCH_2CH_2$ | |
| 1.101 | H | Cl | $CH_2CH_3$ | $CH_2CH_2OCH_2CH_2$ | |
| 1.102 | F | Cl | $CH_2CH_3$ | $CH_2CH_2OCH_2CH_2$ | |
| 1.103 | H | Cl | $CH_2CH_2OCH_3$ | $CH_2CH_2OCH_2CH_2$ | |
| 1.104 | F | Cl | $CH_2CH_2OCH_3$ | $CH_2CH_2OCH_2CH_2$ | |
| 1.105 | H | Cl | $CH_3$ | $CH_2CH_2SCH_2$ | |
| 1.106 | F | Cl | $CH_3$ | $CH_2CH_2SCH_2$ | |
| 1.107 | H | Cl | $CH_2CH_3$ | $CH_2CH_2SCH_2$ | |
| 1.108 | F | Cl | $CH_2CH_3$ | $CH_2CH_2SCH_2$ | |
| 1.109 | H | Cl | $CH_2CH_2OCH_3$ | $CH_2CH_2SCH_2$ | |
| 1.110 | F | Cl | $CH_2CH_2OCH_3$ | $CH_2CH_2SCH_2$ | |
| 1.111 | H | Cl | $CH_3$ | $CH_2CH_2CH_2SCH_2$ | |
| 1.112 | F | Cl | $CH_3$ | $CH_2CH_2CH_2SCH_2$ | |
| 1.113 | H | Cl | $CH_2CH_3$ | $CH_2CH_2CH_2SCH_2$ | |
| 1.114 | F | Cl | $CH_2CH_3$ | $CH_2CH_2CH_2SCH_2$ | |
| 1.115 | H | Cl | $CH_2CH_2OCH_3$ | $CH_2CH_2CH_2SCH_2$ | |
| 1.116 | F | Cl | $CH_2CH_2OCH_3$ | $CH_2CH_2CH_2SCH_2$ | |
| 1.117 | H | Cl | $CH_3$ | $CH_2CH_2CH_2CO$ | Öl |
| 1.118 | F | Cl | $CH_3$ | $CH_2CH_2CH_2CO$ | |
| 1.119 | H | Cl | $CH_2CH_3$ | $CH_2CH_2CH_2CO$ | Öl |
| 1.120 | F | Cl | $CH_2CH_3$ | $CH_2CH_2CH_2CO$ | |
| 1.121 | H | Cl | $CH_2CH_2OCH_3$ | $CH_2CH_2CH_2CO$ | |
| 1.122 | F | Cl | $CH_2CH_2OCH_3$ | $CH_2CH_2CH_2CO$ | |

| Nr. | $R^1$ | $R^2$ | $R^3$ | $R^4$ | $R^5$ | Fp |
|---|---|---|---|---|---|---|
| 1.123 | H | Cl | $CH_3$ | $CH_2CH_2CH_2CH_2CO$ | | |
| 1.124 | F | Cl | $CH_3$ | $CH_2CH_2CH_2CH_2CO$ | | |
| 1.125 | H | Cl | $CH_2CH_3$ | $CH_2CH_2CH_2CH_2CO$ | | 138-140°C |
| 1.126 | F | Cl | $CH_2CH_3$ | $CH_2CH_2CH_2CH_2CO$ | | |
| 1.127 | H | Cl | $CH_2CH_2OCH_3$ | $CH_2CH_2CH_2CH_2CO$ | | |
| 1.128 | F | Cl | $CH_2CH_2OCH_3$ | $CH_2CH_2CH_2CH_2CO$ | | |

Tabelle 2

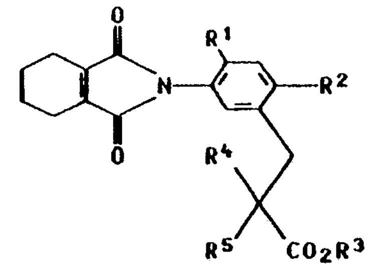

| Nr. | $R^1$ | $R^2$ | $R^3$ | $R^4$ | $R^5$ | Fp |
|---|---|---|---|---|---|---|
| 2.001 | F | Cl | $CH_3$ | H | CN | |
| 2.002 | F | Cl | $CH_2CH_3$ | H | CN | |
| 2.003 | F | Cl | $CH_2CH_2OCH_3$ | H | CN | |
| 2.004 | H | Cl | $CH_3$ | Cl | CN | |
| 2.005 | F | Cl | $CH_3$ | Cl | CN | |
| 2.006 | H | Cl | $CH_2CH_3$ | Cl | CN | |
| 2.007 | F | Cl | $CH_2CH_3$ | Cl | CN | |
| 2.008 | H | Cl | $CH_2CH_2OCH_3$ | Cl | CN | |
| 2.009 | F | Cl | $CH_2CH_2OCH_3$ | Cl | CN | |
| 2.010 | H | Cl | $CH_3$ | Br | CN | |
| 2.011 | F | Cl | $CH_3$ | Br | CN | |
| 2.012 | H | Br | $CH_3$ | Br | CN | |
| 2.013 | F | Br | $CH_3$ | Br | CN | |
| 2.014 | H | Cl | $CH_2CH_3$ | Br | CN | |
| 2.015 | F | Cl | $CH_2CH_3$ | Br | CN | |
| 2.016 | H | Cl | $CH_2CH_2OCH_3$ | Br | CN | |
| 2.017 | F | Cl | $CH_2CH_2OCH_3$ | Br | CN | |
| 2.018 | H | Cl | $CH_3$ | $CH_3$ | CN | |
| 2.019 | F | Cl | $CH_3$ | $CH_3$ | CN | |

| Nr. | $R^1$ | $R^2$ | $R^3$ | $R^4$ | $R^5$ | Fp |
|---|---|---|---|---|---|---|
| 2.020 | H | Cl | $CH_2CH_3$ | $CH_3$ | CN | 111-113 °C |
| 2.021 | F | Cl | $CH_2CH_3$ | $CH_3$ | CN | Öl |
| 2.022 | H | Cl | $CH_2CH_2OCH_3$ | $CH_3$ | CN | |
| 2.023 | F | Cl | $CH_2CH_2OCH_3$ | $CH_3$ | CN | |
| 2.024 | H | Cl | $CH_3$ | $CH_2CH_3$ | CN | |
| 2.025 | F | Cl | $CH_3$ | $CH_2CH_3$ | CN | |
| 2.026 | H | Cl | $CH_2CH_3$ | $CH_2CH_3$ | CN | 124- 6 °C |
| 2.027 | F | Cl | $CH_2CH_3$ | $CH_2CH_3$ | CN | 87- 89°C |
| 2.028 | H | Cl | $CH_2CH_2OCH_3$ | $CH_2CH_3$ | CN | |
| 2.029 | F | Cl | $CH_2CH_2OCH_3$ | $CH_2CH_3$ | CN | |
| 2.030 | H | Cl | $CH_3$ | $CH_2CH_2CH_3$ | CN | |
| 2.031 | F | Cl | $CH_3$ | $CH_2CH_2CH_3$ | CN | |
| 2.032 | H | Cl | $CH_2CH_3$ | $CH_2CH_2CH_3$ | CN | |
| 2.033 | F | Cl | $CH_2CH_3$ | $CH_2CH_2CH_3$ | CN | |
| 2.034 | H | Cl | $CH_2CH_2OCH_3$ | $CH_2CH_2CH_3$ | CN | |
| 2.035 | F | Cl | $CH_2CH_2OCH_3$ | $CH_2CH_2CH_3$ | CN | |
| 2.036 | H | Cl | $CH_3$ | $CH_2CH_2CH_2CH_3$ | CN | 113- 4 °C |
| 2.037 | F | Cl | $CH_3$ | $CH_2CH_2CH_2CH_3$ | CN | 124-125°C |
| 2.038 | H | Cl | $CH_2CH_3$ | $CH_2CH_2CH_2CH_3$ | CN | |
| 2.039 | F | Cl | $CH_2CH_3$ | $CH_2CH_2CH_2CH_3$ | CN | |
| 2.040 | H | Cl | $CH_2CH_2OCH_3$ | $CH_2CH_2CH_2CH_3$ | CN | |
| 2.041 | F | Cl | $CH_2CH_2OCH_3$ | $CH_2CH_2CH_2CH_3$ | CN | |
| 2.042 | H | Cl | $CH_3$ | H | $COCH_3$ | |
| 2.043 | F | Cl | $CH_3$ | H | $COCH_3$ | |
| 2.044 | H | Cl | $CH_2CH_3$ | H | $COCH_3$ | |
| 2.045 | F | Cl | $CH_2CH_3$ | H | $COCH_3$ | |

| Nr. | $R^1$ | $R^2$ | $R^3$ | $R^4$ | $R^5$ | Fp |
|---|---|---|---|---|---|---|
| 2.046 | H | Cl | $CH_2CH_2OCH_3$ | H | $COCH_3$ | |
| 2.047 | F | Cl | $CH_2CH_2OCH_3$ | H | $COCH_3$ | |
| 2.048 | H | Cl | $CH_3$ | Cl | $COCH_3$ | |
| 2.049 | F | Cl | $CH_3$ | Cl | $COCH_3$ | |
| 2.050 | H | Cl | $CH_2CH_3$ | Cl | $COCH_3$ | 123- 5 °C |
| 2.051 | F | Cl | $CH_2CH_3$ | Cl | $COCH_3$ | |
| 2.052 | H | Cl | $CH_2CH_2OCH_3$ | Cl | $COCH_3$ | |
| 2.053 | F | Cl | $CH_2CH_2OCH_3$ | Cl | $COCH_3$ | |
| 2.054 | H | Cl | $CH_3$ | Br | $COCH_3$ | |
| 2.055 | F | Cl | $CH_3$ | Br | $COCH_3$ | |
| 2.056 | H | Br | $CH_3$ | Br | $COCH_3$ | |
| 2.057 | F | Br | $CH_3$ | Br | $COCH_3$ | |
| 2.058 | H | Cl | $CH_2CH_3$ | Br | $COCH_3$ | |
| 2.059 | F | Cl | $CH_2CH_3$ | Br | $COCH_3$ | |
| 2.060 | H | Cl | $CH_2CH_2OCH_3$ | Br | $COCH_3$ | |
| 2.061 | F | Cl | $CH_2CH_2OCH_3$ | Br | $COCH_3$ | |
| 2.062 | H | Cl | $CH_3$ | $CH_3$ | $COCH_3$ | 108-110 °C |
| 2.063 | F | Cl | $CH_3$ | $CH_3$ | $COCH_3$ | Öl |
| 2.064 | H | Cl | $CH_2CH_3$ | $CH_3$ | $COCH_3$ | 103- 4 °C |
| 2.065 | F | Cl | $CH_2CH_3$ | $CH_3$ | $COCH_3$ | Öl |
| 2.066 | H | Cl | $CH_2CH_2OCH_3$ | $CH_3$ | $COCH_3$ | |
| 2.067 | F | Cl | $CH_2CH_2OCH_3$ | $CH_3$ | $COCH_3$ | |
| 2.068 | H | Cl | $CH_3$ | $CH_2CH_3$ | $COCH_3$ | |
| 2.069 | F | Cl | $CH_3$ | $CH_2CH_3$ | $COCH_3$ | |
| 2.070 | H | Cl | $CH_2CH_3$ | $CH_2CH_3$ | $COCH_3$ | Ol |
| 2.071 | F | Cl | $CH_2CH_3$ | $CH_2CH_3$ | $COCH_3$ | Ol |

| Nr. | $R^1$ | $R^2$ | $R^3$ | $R^4$ | $R^5$ | Fp |
|---|---|---|---|---|---|---|
| 2.072 | H | Cl | $CH_2CH_2OCH_3$ | $CH_2CH_3$ | $COCH_3$ | |
| 2.073 | F | Cl | $CH_2CH_2OCH_3$ | $CH_2CH_3$ | $COCH_3$ | |
| 2.074 | H | Cl | $CH_3$ | H | $COCH_2CH_3$ | |
| 2.075 | F | Cl | $CH_3$ | H | $COCH_2CH_3$ | |
| 2.076 | H | Cl | $CH_2CH_3$ | H | $COCH_2CH_3$ | |
| 2.077 | F | Cl | $CH_2CH_3$ | H | $COCH_2CH_3$ | |
| 2.078 | H | Cl | $CH_2CH_2OCH_3$ | H | $COCH_2CH_3$ | |
| 2.079 | F | Cl | $CH_2CH_2OCH_3$ | H | $COCH_2CH_3$ | |
| 2.080 | H | Cl | $CH_3$ | Cl | $COCH_2CH_3$ | |
| 2.081 | F | Cl | $CH_3$ | Cl | $COCH_2CH_3$ | |
| 2.082 | H | Cl | $CH_2CH_3$ | Cl | $COCH_2CH_3$ | |
| 2.083 | F | Cl | $CH_2CH_3$ | Cl | $COCH_2CH_3$ | |
| 2.084 | H | Cl | $CH_2CH_2OCH_3$ | Cl | $COCH_2CH_3$ | |
| 2.085 | F | Cl | $CH_2CH_2OCH_3$ | Cl | $COCH_2CH_3$ | |
| 2.086 | H | Cl | $CH_3$ | Br | $COCH_2CH_3$ | |
| 2.087 | F | Cl | $CH_3$ | Br | $COCH_2CH_3$ | |
| 2.088 | H | Br | $CH_3$ | Br | $COCH_2CH_3$ | |
| 2.089 | F | Br | $CH_3$ | Br | $COCH_2CH_3$ | |
| 2.090 | H | Cl | $CH_2CH_3$ | Br | $COCH_2CH_3$ | |
| 2.091 | F | Cl | $CH_2CH_3$ | Br | $COCH_2CH_3$ | |
| 2.092 | H | Cl | $CH_2CH_2OCH_3$ | Br | $COCH_2CH_3$ | |
| 2.093 | F | Cl | $CH_2CH_2OCH_3$ | Br | $COCH_2CH_3$ | |
| 2.094 | H | Cl | $CH_3$ | $CH_3$ | $COCH_2CH_3$ | |
| 2.095 | F | Cl | $CH_3$ | $CH_3$ | $COCH_2CH_3$ | |
| 2.096 | H | Cl | $CH_2CH_3$ | $CH_3$ | $COCH_2CH_3$ | |
| 2.097 | F | Cl | $CH_2CH_3$ | $CH_3$ | $COCH_2CH_3$ | |

| Nr. | $R^1$ | $R^2$ | $R^3$ | $R^4$ | $R^5$ | Fp |
|---|---|---|---|---|---|---|
| 2.098 | H | Cl | $CH_2CH_2OCH_3$ | $CH_3$ | $COCH_2CH_3$ | |
| 2.099 | F | Cl | $CH_2CH_2OCH_3$ | $CH_3$ | $COCH_2CH_3$ | |
| 2.100 | H | Cl | $CH_3$ | $CH_2CH_3$ | $COCH_2CH_3$ | |
| 2.101 | F | Cl | $CH_3$ | $CH_2CH_3$ | $COCH_2CH_3$ | |
| 2.102 | H | Cl | $CH_2CH_3$ | $CH_2CH_3$ | $COCH_2CH_3$ | |
| 2.103 | F | Cl | $CH_2CH_3$ | $CH_2CH_3$ | $COCH_2CH_3$ | |
| 2.104 | H | Cl | $CH_2CH_2OCH_3$ | $CH_2CH_3$ | $COCH_2CH_3$ | |
| 2.105 | F | Cl | $CH_2CH_2OCH_3$ | $CH_2CH_3$ | $COCH_2CH_3$ | |
| 2.106 | H | Cl | $CH_3$ | $CH_2CH_2CH_3$ | $COCH_3$ | |
| 2.107 | F | Cl | $CH_3$ | $CH_2CH_2CH_3$ | $COCH_3$ | |
| 2.108 | H | Cl | $CH_3$ | $CH_2CH_2CH_2CH_3$ | $COCH_3$ | 117-119°C |
| 2.109 | F | Cl | $CH_3$ | $CH_2CH_2CH_2CH_3$ | $COCH_3$ | 84- 86°C |
| 2.110 | H | Cl | $CH_3$ | $CH_3$ | $COCH(CH_3)_2$ | 120-122°C |
| 2.111 | F | Cl | $CH_3$ | $CH_3$ | $COCH(CH_3)_2$ | Öl |

Tabelle 3

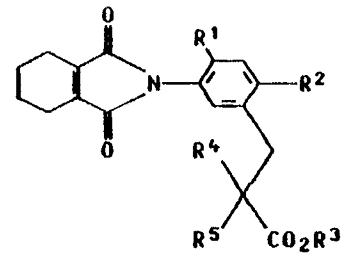

| Nr. | $R^1$ | $R^2$ | $R^3$ | $R^4$ | $R^5$ | Fp |
|---|---|---|---|---|---|---|
| 3.001 | F | Cl | $CH_3$ | H | $CO_2CH_3$ | 115-117 °C |
| 3.002 | H | Cl | $CH_3$ | Cl | $CO_2CH_3$ | 131-133°C |
| 3.003 | F | Cl | $CH_3$ | Cl | $CO_2CH_3$ | 122-124°C |
| 3.004 | H | Br | $CH_3$ | Cl | $CO_2CH_3$ | |
| 3.005 | F | Br | $CH_3$ | Cl | $CO_2CH_3$ | |
| 3.006 | H | Br | $CH_3$ | Br | $CO_2CH_3$ | |
| 3.007 | F | Br | $CH_3$ | Br | $CO_2CH_3$ | |
| 3.008 | H | Cl | $CH_3$ | Br | $CO_2CH_3$ | 102-106°C |
| 3.009 | F | Cl | $CH_3$ | Br | $CO_2CH_3$ | |
| 3.010 | H | Cl | $CH_3$ | $CH_3$ | $CO_2CH_3$ | 103-105°C |
| 3.011 | F | Cl | $CH_3$ | $CH_3$ | $CO_2CH_3$ | |
| 3.012 | H | Cl | $CH_3$ | $CH_2CH_3$ | $CO_2CH_3$ | |
| 3.013 | F | Cl | $CH_3$ | $CH_2CH_3$ | $CO_2CH_3$ | |
| 3.014 | H | Cl | $CH_3$ | $CH_2CH_2CH_3$ | $CO_2CH_3$ | 128-130°C |
| 3.015 | F | Cl | $CH_3$ | $CH_2CH_2CH_3$ | $CO_2CH_3$ | |
| 3.016 | H | Cl | $CH_3$ | $CH_2CH(CH_3)_2$ | $CO_2CH_3$ | |
| 3.017 | F | Cl | $CH_3$ | $CH_2CH(CH_3)_2$ | $CO_2CH_3$ | |
| 3.018 | H | Cl | $CH_3$ | $CH_2$- 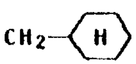 | $CO_2CH_3$ | Öl |
| 3.019 | F | Cl | $CH_3$ | $CH_2$- 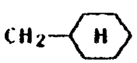 | $CO_2CH_3$ | |

| Nr. | $R^1$ | $R^2$ | $R^3$ | $R^4$ | $R^5$ | Fp |
|---|---|---|---|---|---|---|
| 3.020 | H | Cl | $CH_3$ | 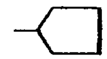 | $CO_2CH_3$ | |
| 3.021 | F | Cl | $CH_3$ | 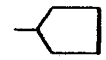 | $CO_2CH_3$ | |
| 3.022 | H | Cl | $CH_3$ | 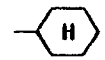 | $CO_2CH_3$ | |
| 3.023 | F | Cl | $CH_3$ | 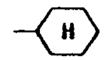 | $CO_2CH_3$ | |
| 3.024 | H | Cl | $CH_3$ | $CH_2CH{=}CH_2$ | $CO_2CH_3$ | 108-110°C |
| 3.025 | F | Cl | $CH_3$ | $CH_2CH{=}CH_2$ | $CO_2CH_3$ | |
| 3.026 | H | Cl | $CH_3$ | $CH_2CH{=}CHCH_3$ | $CO_2CH_3$ | 90- 93°C |
| 3.027 | F | Cl | $CH_3$ | $CH_2CH{=}CHCH_3$ | $CO_2CH_3$ | |
| 3.028 | H | Cl | $CH_3$ | $CH_2CH_2CH{=}CH_2$ | $CO_2CH_3$ | 100-102°C |
| 3.029 | F | Cl | $CH_3$ | $CH_2CH_2CH{=}CH_2$ | $CO_2CH_3$ | |
| 3.030 | H | Cl | $CH_3$ | $CH_2C{\equiv}CH$ | $CO_2CH_3$ | 133-135°C |
| 3.031 | F | Cl | $CH_3$ | $CH_2C{\equiv}CH$ | $CO_2CH_3$ | |
| 3.032 | H | Cl | $CH_3$ | $CH_2C{\equiv}CCH_3$ | $CO_2CH_3$ | |
| 3.033 | F | Cl | $CH_3$ | $CH_2C{\equiv}CCH_3$ | $CO_2CH_3$ | |
| 3.034 | H | Cl | $CH_3$ | $CH_2CH_2OCH_3$ | $CO_2CH_3$ | Öl |
| 3.035 | F | Cl | $CH_3$ | $CH_2CH_2OCH_3$ | $CO_2CH_3$ | |
| 3.036 | H | Cl | $CH_3$ | $CH_2CH_2OCH_2CH_3$ | $CO_2CH_3$ | Öl |
| 3.037 | F | Cl | $CH_3$ | $CH_2CH_2OCH_2CH_3$ | $CO_2CH_3$ | |
| 3.038 | H | Cl | $CH_3$ | $CH_2CH_2SCH_3$ | $CO_2CH_3$ | |
| 3.039 | F | Cl | $CH_3$ | $CH_2CH_2SCH_3$ | $CO_2CH_3$ | |
| 3.040 | H | Cl | $CH_3$ | $CH_2CH_2SCH_2CH_3$ | $CO_2CH_3$ | |
| 3.041 | F | Cl | $CH_3$ | $CH_2CH_2SCH_2CH_3$ | $CO_2CH_3$ | |
| 3.042 | F | Cl | $CH_2CH_3$ | H | $CO_2CH_2CH_3$ | 89-91 °C |

| Nr. | $R^1$ | $R^2$ | $R^3$ | $R^4$ | $R^5$ | Fp |
|---|---|---|---|---|---|---|
| 3.043 | H | Cl | $CH_2CH_3$ | Cl | $CO_2CH_2CH_3$ | |
| 3.044 | F | Cl | $CH_2CH_3$ | Cl | $CO_2CH_2CH_3$ | |
| 3.045 | H | Br | $CH_2CH_3$ | Cl | $CO_2CH_2CH_3$ | |
| 3.046 | F | Br | $CH_2CH_3$ | Cl | $CO_2CH_2CH_3$ | |
| 3.047 | H | Br | $CH_2CH_3$ | Br | $CO_2CH_2CH_3$ | |
| 3.048 | F | Br | $CH_2CH_3$ | Br | $CO_2CH_2CH_3$ | |
| 3.049 | H | Cl | $CH_2CH_3$ | Br | $CO_2CH_2CH_3$ | |
| 3.050 | F | Cl | $CH_2CH_3$ | Br | $CO_2CH_2CH_3$ | |
| 3.051 | H | Cl | $CH_2CH_3$ | $CH_3$ | $CO_2CH_2CH_3$ | |
| 3.052 | F | Cl | $CH_2CH_3$ | $CH_3$ | $CO_2CH_2CH_3$ | |
| 3.053 | H | Cl | $CH_2CH_3$ | $CH_2CH_3$ | $CO_2CH_2CH_3$ | |
| 3.054 | F | Cl | $CH_2CH_3$ | $CH_2CH_3$ | $CO_2CH_2CH_3$ | |
| 3.055 | H | Cl | $CH_2CH_3$ | $CH_2CH_2CH_3$ | $CO_2CH_2CH_3$ | |
| 3.056 | F | Cl | $CH_2CH_3$ | $CH_2CH_2CH_3$ | $CO_2CH_2CH_3$ | |
| 3.057 | H | Cl | $CH_2CH_3$ | $CH_2CH(CH_3)_2$ | $CO_2CH_2CH_3$ | |
| 3.058 | F | Cl | $CH_2CH_3$ | $CH_2CH(CH_3)_2$ | $CO_2CH_2CH_3$ | |
| 3.059 | H | Cl | $CH_2CH_3$ | 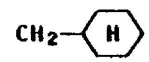 | $CO_2CH_2CH_3$ | |
| 3.060 | F | Cl | $CH_2CH_3$ | 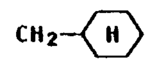 | $CO_2CH_2CH_3$ | |
| 3.061 | H | Cl | $CH_2CH_3$ | 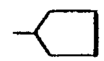 | $CO_2CH_2CH_3$ | |
| 3.062 | F | Cl | $CH_2CH_3$ | 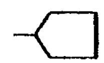 | $CO_2CH_2CH_3$ | |
| 3.063 | H | Cl | $CH_2CH_3$ | 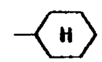 | $CO_2CH_2CH_3$ | |
| 3.064 | F | Cl | $CH_2CH_3$ | 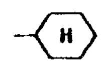 | $CO_2CH_2CH_3$ | |
| 3.065 | H | Cl | $CH_2CH_3$ | $CH_2CH=CH_2$ | $CO_2CH_2CH_3$ | |
| 3.066 | F | Cl | $CH_2CH_3$ | $CH_2CH=CH_2$ | $CO_2CH_2CH_3$ | |

| Nr. | $R^1$ | $R^2$ | $R^3$ | $R^4$ | $R^5$ | Fp |
|---|---|---|---|---|---|---|
| 3.067 | H | Cl | $CH_2CH_3$ | $CH_2CH=CHCH_3$ | $CO_2CH_2CH_3$ | |
| 3.068 | F | Cl | $CH_2CH_3$ | $CH_2CH=CHCH_3$ | $CO_2CH_2CH_3$ | |
| 3.069 | H | Cl | $CH_2CH_3$ | $CH_2CH_2CH=CH_2$ | $CO_2CH_2CH_3$ | |
| 3.070 | F | Cl | $CH_2CH_3$ | $CH_2CH_2CH=CH_2$ | $CO_2CH_2CH_3$ | |
| 3.071 | H | Cl | $CH_2CH_3$ | $CH_2C\equiv CH$ | $CO_2CH_2CH_3$ | |
| 3.072 | F | Cl | $CH_2CH_3$ | $CH_2C\equiv CH$ | $CO_2CH_2CH_3$ | |
| 3.073 | H | Cl | $CH_2CH_3$ | $CH_2C\equiv CCH_3$ | $CO_2CH_2CH_3$ | |
| 3.074 | F | Cl | $CH_2CH_3$ | $CH_2C\equiv CCH_3$ | $CO_2CH_3CH_3$ | |
| 3.075 | H | Cl | $CH_2CH_3$ | $CH_2CH_2OCH_3$ | $CO_2CH_2CH_3$ | |
| 3.076 | F | Cl | $CH_2CH_3$ | $CH_2CH_2OCH_3$ | $CO_2CH_2CH_3$ | |
| 3.077 | H | Cl | $CH_2CH_3$ | $CH_2CH_2OCH_2CH_3$ | $CO_2CH_2CH_3$ | |
| 3.078 | F | Cl | $CH_2CH_3$ | $CH_2CH_2OCH_2CH_3$ | $CO_2CH_2CH_3$ | |
| 3.079 | H | Cl | $CH_2CH_3$ | $CH_2CH_2SCH_3$ | $CO_2CH_2CH_3$ | |
| 3.080 | F | Cl | $CH_2CH_3$ | $CH_2CH_2SCH_3$ | $CO_2CH_2CH_3$ | |
| 3.081 | H | Cl | $CH_2CH_3$ | $CH_2CH_2SCH_2CH_3$ | $CO_2CH_2CH_3$ | |
| 3.082 | F | Cl | $CH_2CH_3$ | $CH_2CH_2SCH_2CH_3$ | $CO_2CH_2CH_3$ | |
| 3.083 | F | Cl | $CH_2CH_2CH_3$ | H | $CO_2CH_2CH_2CH_3$ | |
| 3.084 | H | Cl | $CH_2CH_2CH_3$ | Cl | $CO_2CH_2CH_2CH_3$ | |
| 3.085 | F | Cl | $CH_2CH_2CH_3$ | Cl | $CO_2CH_2CH_2CH_3$ | |
| 3.086 | H | Br | $CH_2CH_2CH_3$ | Cl | $CO_2CH_2CH_2CH_3$ | |
| 3.087 | F | Br | $CH_2CH_2CH_3$ | Cl | $CO_2CH_2CH_2CH_3$ | |
| 3.088 | H | Br | $CH_2CH_2CH_3$ | Br | $CO_2CH_2CH_2CH_3$ | |
| 3.089 | F | Br | $CH_2CH_2CH_3$ | Br | $CO_2CH_2CH_2CH_3$ | |
| 3.090 | H | Cl | $CH_2CH_2CH_3$ | Br | $CO_2CH_2CH_2CH_3$ | |
| 3.091 | F | Cl | $CH_2CH_2CH_3$ | Br | $CO_2CH_2CH_2CH_3$ | |
| 3.092 | H | Cl | $CH_2CH_2CH_3$ | $CH_3$ | $CO_2CH_2CH_2CH_3$ | |
| 3.093 | F | Cl | $CH_2CH_2CH_3$ | $CH_3$ | $CO_2CH_2CH_2CH_3$ | |
| 3.094 | H | Cl | $CH_2CH_2CH_3$ | $CH_2CH_3$ | $CO_2CH_2CH_2CH_3$ | |
| 3.095 | F | Cl | $CH_2CH_2CH_3$ | $CH_2CH_3$ | $CO_2CH_2CH_2CH_3$ | |
| 3.096 | H | Cl | $CH_2CH_2CH_3$ | $CH_2CH_2CH_3$ | $CO_2CH_2CH_2CH_3$ | |
| 3.097 | F | Cl | $CH_2CH_2CH_3$ | $CH_2CH_2CH_3$ | $CO_2CH_2CH_2CH_3$ | |

| Nr. | $R^1$ | $R^2$ | $R^3$ | $R^4$ | $R^5$ | Fp |
|---|---|---|---|---|---|---|
| 3.098 | H | Cl | $CH_2CH_2CH_3$ | $CH_2CH(CH_3)_2$ | $CO_2CH_2CH_2CH_3$ | |
| 3.099 | F | Cl | $CH_2CH_2CH_3$ | $CH_2CH(CH_3)_2$ | $CO_2CH_2CH_2CH_3$ | |
| 3.100 | H | Cl | $CH_2CH_2CH_3$ | $CH_2$— 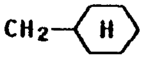 | $CO_2CH_2CH_2CH_3$ | |
| 3.101 | F | Cl | $CH_2CH_2CH_3$ | $CH_2$— 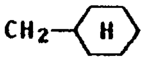 | $CO_2CH_2CH_2CH_3$ | |
| 3.102 | H | Cl | $CH_2CH_2CH_3$ | 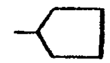 | $CO_2CH_2CH_2CH_3$ | |
| 3.103 | F | Cl | $CH_2CH_2CH_3$ | 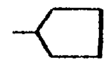 | $CO_2CH_2CH_2CH_3$ | |
| 3.104 | H | Cl | $CH_2CH_2CH_3$ | 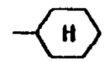 | $CO_2CH_2CH_2CH_3$ | |
| 3.105 | F | Cl | $CH_2CH_2CH_3$ | 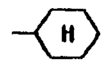 | $CO_2CH_2CH_2CH_3$ | |
| 3.106 | H | Cl | $CH_2CH_2CH_3$ | $CH_2CH=CH_2$ | $CO_2CH_2CH_2CH_3$ | |
| 3.107 | F | Cl | $CH_2CH_2CH_3$ | $CH_2CH=CH_2$ | $CO_2CH_2CH_2CH_3$ | |
| 3.108 | H | Cl | $CH_2CH_2CH_3$ | $CH_2CH=CHCH_3$ | $CO_2CH_2CH_2CH_3$ | |
| 3.109 | F | Cl | $CH_2CH_6CH_3$ | $CH_2CH=CHCH_3$ | $CO_2CH_2CH_2CH_3$ | |
| 3.110 | H | Cl | $CH_2CH_2CH_3$ | $CH_2CH_2CH=CH_2$ | $CO_2CH_2CH_2CH_3$ | |
| 3.111 | F | Cl | $CH_2CH_2CH_3$ | $CH_2CH_2CH=CH_2$ | $CO_2CH_2CH_2CH_3$ | |
| 3.112 | H | Cl | $CH_2CH_2CH_3$ | $CH_2C\equiv CH$ | $CO_2CH_2CH_2CH_3$ | |
| 3.113 | F | Cl | $CH_2CH_2CH_3$ | $CH_2C\equiv CH$ | $CO_2CH_2CH_2CH_3$ | |
| 3.114 | H | Cl | $CH_2CH_2CH_3$ | $CH_2C\equiv CCH_3$ | $CO_2CH_2CH_2CH_3$ | |
| 3.115 | F | Cl | $CH_2CH_2CH_3$ | $CH_2C\equiv CCH_3$ | $CO_2CH_2CH_2CH_3$ | |
| 3.116 | H | Cl | $CH_2CH_2CH_3$ | $CH_2CH_2OCH_3$ | $CO_2CH_2CH_2CH_3$ | |
| 3.117 | F | Cl | $CH_2CH_2CH_3$ | $CH_2CH_2OCH_3$ | $CO_2CH_2CH_2CH_3$ | |
| 3.118 | H | Cl | $CH_2CH_2CH_3$ | $CH_2CH_2OCH_2CH_3$ | $CO_2CH_2CH_2CH_3$ | |
| 3.119 | F | Cl | $CH_2CH_2CH_3$ | $CH_2CH_2OCH_2CH_3$ | $CO_2CH_2CH_2CH_3$ | |

| Nr. | $R^1$ | $R^2$ | $R^3$ | $R^4$ | $R^5$ | Fp |
|---|---|---|---|---|---|---|
| 3.120 | H | Cl | $CH_2CH_2CH_3$ | $CH_2CH_2SCH_3$ | $CO_2CH_2CH_2CH_3$ | |
| 3.121 | F | Cl | $CH_2CH_2CH_3$ | $CH_2CH_2SCH_3$ | $CO_2CH_2CH_2CH_3$ | |
| 3.122 | H | Cl | $CH_2CH_2CH_3$ | $CH_2CH_2SCH_2CH_3$ | $CO_2CH_2CH_2CH_3$ | |
| 3.123 | F | Cl | $CH_2CH_2CH_3$ | $CH_2CH_2SCH_2CH_3$ | $CO_2CH_2CH_2CH_3$ | |
| 3.124 | H | Cl | $CH_3$ | $CH(CH_3)_2$ | $CO_2CH_3$ | |
| 3.125 | F | Cl | $CH_3$ | $CH(CH_3)_2$ | $CO_2CH_3$ | |
| 3.126 | H | Cl | $CH_3$ | $CH_2CH_2CH_2CH_3$ | $CO_2CH_3$ | 99-101°C |
| 3.127 | F | Cl | $CH_3$ | $CH_2CH_2CH_2CH_3$ | $CO_2CH_3$ | |
| 3.128 | H | Cl | $CH_3$ | $CH_2SCH_3$ | $CO_2CH_3$ | 130-133°C |
| 3.129 | F | Cl | $CH_3$ | $CH_2SCH_3$ | $CO_2CH_3$ | |

Tabelle 4

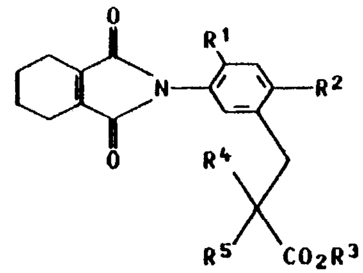

| Nr. | $R^1$ | $R^2$ | $R^3$ | $R^4$ | $R^5$ | Fp |
|---|---|---|---|---|---|---|
| 4.001 | H | Cl | | $CH_2CH_2$ | CN | |
| 4.002 | F | Cl | | $CH_2CH_2$ | CN | |
| 4.003 | H | Cl | | $CH_2CH_2CH_2$ | CN | |
| 4.004 | F | Cl | | $CH_2CH_2CH_2$ | CN | |
| 4.005 | H | Cl | | $CH_2CH_2CH_2CH_2$ | CN | |
| 4.006 | F | Cl | | $CH_2CH_2CH_2CH_2$ | CN | |
| 4.007 | H | Cl | | $CH_2CH_2$ | $CO_2CH_3$ | |
| 4.008 | F | Cl | | $CH_2CH_2$ | $CO_2CH_3$ | |
| 4.009 | H | Cl | | $CH_2CH_2CH_2$ | $CO_2CH_3$ | |
| 4.010 | F | Cl | | $CH_2CH_2CH_2$ | $CO_2CH_3$ | |
| 4.011 | H | Cl | | $CH_2CH_2CH_2CH_2$ | $CO_2CH_3$ | |
| 4.012 | F | Cl | | $CH_2CH_2CH_2CH_2$ | $CO_2CH_3$ | |
| 4.013 | H | Cl | | $CH_2CH_2$ | $CO_2CH_2CH_3$ | |
| 4.014 | F | Cl | | $CH_2CH_2$ | $CO_2CH_2CH_3$ | |
| 4.015 | H | Cl | | $CH_2CH_2CH_2$ | $CO_2CH_2CH_3$ | |
| 4.016 | F | Cl | | $CH_2CH_2CH_2$ | $CO_2CH_2CH_3$ | |
| 4.017 | H | Cl | | $CH_2CH_2CH_2CH_2$ | $CO_2CH_2CH_3$ | |
| 4.018 | F | Cl | | $CH_2CH_2CH_2CH_2$ | $CO_2CH_2CH_3$ | |
| 4.019 | H | Cl | | $CH_2CH_2$ | $CO_2CH_2CH_2OCH_3$ | |

| Nr. | $R^1$ | $R^2$ | $R^3$ | $R^4$ | $R^5$ | Fp |
|---|---|---|---|---|---|---|
| 4.020 | F | Cl | | $CH_2CH_2$ | $CO_2CH_2CH_2OCH_3$ | |
| 4.021 | H | Cl | | $CH_2CH_2CH_2$ | $CO_2CH_2CH_2OCH_3$ | |
| 4.022 | F | Cl | | $CH_2CH_2CH_2$ | $CO_2CH_2CH_2OCH_3$ | |
| 4.023 | H | Cl | | $CH_2CH_2CH_2CH_2$ | $CO_2CH_2CH_2OCH_3$ | |
| 4.024 | F | Cl | | $CH_2CH_2CH_2CH_2$ | $CO_2CH_2CH_2OCH_3$ | |
| 4.025 | H | Cl | | $CH_2CH_2$ | $COCH_3$ | 167-169°C |
| 4.026 | F | Cl | | $CH_2CH_2$ | $COCH_3$ | |
| 4.027 | H | Cl | | $CH_2CH_2CH_2$ | $COCH_3$ | |
| 4.028 | F | Cl | | $CH_2CH_2CH_2$ | $COCH_3$ | |
| 4.029 | H | Cl | | $CH_2CH_2CH_2CH_2$ | $COCH_3$ | |
| 4.030 | F | Cl | | $CH_2CH_2CH_2CH_2$ | $COCH_3$ | |
| 4.031 | H | Cl | | $CH_2CH_2$ | $COCH_2CH_3$ | 106-108°C |
| 4.032 | F | Cl | | $CH_2CH_2$ | $COCH_2CH_3$ | |
| 4.033 | H | Cl | | $CH_2CH_2CH_2$ | $COCH_2CH_3$ | |
| 4.034 | F | Cl | | $CH_2CH_2CH_2$ | $COCH_2CH_3$ | |
| 4.035 | H | Cl | | $CH_2CH_2CH_2CH_2$ | $COCH_2CH_3$ | |
| 4.036 | F | Cl | | $CH_2CH_2CH_2CH_2$ | $COCH_2CH_3$ | |
| 4.037 | H | Cl | | $CH_2CH_2$ | $CH_3$ | |
| 4.038 | F | Cl | | $CH_2CH_2$ | $CH_3$ | |
| 4.039 | H | Cl | | $CH_2CH_2CH_2$ | $CH_3$ | |
| 4.040 | F | Cl | | $CH_2CH_2CH_2$ | $CH_3$ | |
| 4.041 | H | Cl | | $CH_2CH_2CH_2CH_2$ | $CH_3$ | |
| 4.042 | F | Cl | | $CH_2CH_2CH_2CH_2$ | $CH_3$ | |
| 4.043 | H | Cl | | $CH_2CH_2$ | $CH_2CH_3$ | |
| 4.044 | F | Cl | | $CH_2CH_2$ | $CH_2CH_3$ | |
| 4.045 | H | Cl | | $CH_2CH_2CH_2$ | $CH_2CH_3$ | |

| Nr. | $R^1$ | $R^2$ | $R^3$ | $R^4$ | $R^5$ | Fp |
|---|---|---|---|---|---|---|
| 4.046 | F | Cl | | $CH_2CH_2CH_2$ | $CH_2CH_3$ | |
| 4.047 | H | Cl | | $CH_2CH_2CH_2CH_2$ | $CH_2CH_3$ | |
| 4.048 | F | Cl | | $CH_2CH_2CH_2CH_2$ | $CH_2CH_3$ | |
| 4.049 | H | Cl | | $CH_2CH_2$ | $CH_2CH_2CH_3$ | |
| 4.050 | F | Cl | | $CH_2CH_2$ | $CH_2CH_2CH_3$ | |
| 4.051 | H | Cl | | $CH_2CH_2CH_2$ | $CH_2CH_2CH_3$ | |
| 4.052 | F | Cl | | $CH_2CH_2CH_2$ | $CH_2CH_2CH_3$ | |
| 4.053 | H | Cl | | $CH_2CH_2CH_2CH_2$ | $CH_2CH_2CH_3$ | |
| 4.054 | F | Cl | | $CH_2CH_2CH_2CH_2$ | $CH_2CH_2CH_3$ | |
| 4.055 | H | Cl | | $CH_2CH_2$ | $CH_2CH_2OCH_3$ | |
| 4.056 | F | Cl | | $CH_2CH_2$ | $CH_2CH_2OCH_3$ | |
| 4.057 | H | Cl | | $CH_2CH_2CH_2$ | $CH_2CH_2OCH_3$ | |
| 4.058 | F | Cl | | $CH_2CH_2CH_2$ | $CH_2CH_2OCH_3$ | |
| 4.059 | H | Cl | | $CH_2CH_2CH_2CH_2$ | $CH_2CH_2OCH_3$ | |
| 4.060 | F | Cl | | $CH_2CH_2CH_2CH_2$ | $CH_2CH_2OCH_3$ | |
| 4.061 | H | Cl | | $CH_2CH_2$ | $CH_2CH_2OCH_2CH_3$ | |
| 4.062 | F | Cl | | $CH_2CH_2$ | $CH_2CH_2OCH_2CH_3$ | |
| 4.063 | H | Cl | | $CH_2CH_2CH_2$ | $CH_2CH_2OCH_2CH_3$ | |
| 4.064 | F | Cl | | $CH_2CH_2CH_2$ | $CH_2CH_2OCH_2CH_3$ | |
| 4.065 | H | Cl | | $CH_2CH_2CH_2CH_2$ | $CH_2CH_2OCH_2CH_3$ | |
| 4.066 | F | Cl | | $CH_2CH_2CH_2CH_2$ | $CH_2CH_2OCH_2CH_3$ | |
| 4.067 | H | Cl | | $CH_2CH_2$ | $CH_2CH_2SCH_3$ | |
| 4.068 | F | Cl | | $CH_2CH_2$ | $CH_2CH_2SCH_3$ | |
| 4.069 | H | Cl | | $CH_2CH_2CH_2$ | $CH_2CH_2SCH_3$ | |
| 4.070 | F | Cl | | $CH_2CH_2CH_2$ | $CH_2CH_2SCH_3$ | |
| 4.071 | H | Cl | | $CH_2CH_2CH_2CH_2$ | $CH_2CH_2SCH_3$ | |

| Nr. | $R^1$ | $R^2$ | $R^3$ | $R^4$ | $R^5$ | Fp |
|---|---|---|---|---|---|---|
| 4.072 | F | Cl | | $CH_2CH_2CH_2CH_2$ | $CH_2CH_2SCH_3$ | |
| 4.073 | H | Cl | | $CH_2CH_2$ | $CH_2CH=CH_2$ | |
| 4.074 | F | Cl | | $CH_2CH_2$ | $CH_2CH=CH_2$ | |
| 4.075 | H | Cl | | $CH_2CH_2CH_2$ | $CH_2CH=CH_2$ | |
| 4.076 | F | Cl | | $CH_2CH_2CH_2$ | $CH_2CH=CH_2$ | |
| 4.077 | H | Cl | | $CH_2CH_2CH_2CH_2$ | $CH_2CH=CH_2$ | |
| 4.078 | F | Cl | | $CH_2CH_2CH_2CH_2$ | $CH_2CH=CH_2$ | |
| 4.079 | H | Cl | | $CH_2CH_2$ | $CH_2CH=CHCH_3$ | |
| 4.080 | F | Cl | | $CH_2CH_2$ | $CH_2CH=CHCH_3$ | |
| 4.081 | H | Cl | | $CH_2CH_2CH_2$ | $CH_2CH=CHCH_3$ | |
| 4.082 | F | Cl | | $CH_2CH_2CH_2$ | $CH_2CH=CHCH_3$ | |
| 4.083 | H | Cl | | $CH_2CH_2CH_2CH_2$ | $CH_2CH=CHCH_3$ | |
| 4.084 | F | Cl | | $CH_2CH_2CH_2CH_2$ | $CH_2CH=CHCH_3$ | |
| 4.085 | H | Cl | | $CH_2CH_2$ | $CH_2C\equiv CH$ | |
| 4.086 | F | Cl | | $CH_2CH_2$ | $CH_2C\equiv CH$ | |
| 4.087 | H | Cl | | $CH_2CH_2CH_2$ | $CH_2C\equiv CH$ | |
| 4.088 | F | Cl | | $CH_2CH_2CH_2$ | $CH_2C\equiv CH$ | |
| 4.089 | H | Cl | | $CH_2CH_2CH_2CH_2$ | $CH_2C\equiv CH$ | |
| 4.090 | F | Cl | | $CH_2CH_2CH_2CH_2$ | $CH_2C\equiv CH$ | |

Die Verbindungen der Formel I haben, wie eingangs angegeben, eine herbizide Wirkrung und sind gegenüber Kulturpflanzen selektiv.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und

Wachstumsstadien 0,01 bis 5,0, vorzugsweise 0,03 bis 0,5 kg/ha.

Die herbizide Wirkung der erfindungsgemäßen Verbindungen auf das Wachstum der Testpflanzen wird durch folgende Gewächshausversuche gezeigt.

Als Kulturgefäße dienen Plastikblumentöpfe mit 300 $cm^3$ Inhalt und lehmigem Sand mit etwa 3.0 % Humus als Substrat. Die Samen der Testpflanzen werden nach Arten getrennt eingesät.

Zum Zweck der Nachauflaufbehandlung zieht man die Testpflanzen je nach Wuchsform erst bis zu einer Wuchshöhe von 3 bis 15 cm an und behandelt sie dann mit den in Wasser als Verteilungsmittel suspendierten oder emulgierten Wirkstoffen, die mittels fein verteilender Düsen gespritzt werden. Es werden direkt gesäte und in den gleichen Gefäßen aufgewachsene Pflanzen ausgewählt, oder sie werden erst als Keimpflanzen getrennt angezogen. Die Aufwandmenge für die Nachauflaufbehandlung beträgt 0.5 kg a.S./ha. Eine Abdeckung unterbleibt bei der Nachauflaufbehandlung.

Die Versuchsgefäße werden im Gewächshausversuch aufgestellt, wobei für wärmeliebende Arten wärmere Bereiche (20 bis 36 °C) und für solche gemäßigter Klimate 10 bis 20 °C bevorzugt werden. Die Versuchsperiode erstreckt sich über 2 bis 4 Wochen. Während dieser Zeit werden die Pflanzen gepflegt und ihre Reaktion auf die einzelnen Behandlungen wird ausgewertet.

Bewertet wird nach einer Skala von 0 bis 100. Dabei bedeutet 100 kein Aufgehen der Sämlinge bzw. völlige Zerstörung zumindest der oberirdischen Teile.

In den Gewächshausversuchen wurden folgende Arten verwendet:

| Abkürz. | Lateinischer Name | Deutscher Name |
|---|---|---|
| AMARE | Amaranthus retroflexus | Zurückgekrümmter Fuchsschwanz |
| CASTO | Cassia tora | - |
| CENCY | Centaurea cyanus | Kornblume |
| ECHGG | Echinochloa grus-galli | Hühnerhirse |
| GALAP | Galium aparine | Klettenlabkraut |
| IPOSS | Ipomoea spp. | Prunkwindearten |
| MERAN | Mercurialis annua | Einjähriges Bingelkraut |
| VIOSS | Viola spp. | Stiefmütterchen |

Die Tabellen A, B und D zeigen, daß die Beispielwirkstoffe 1.058, 1,046, 1.032, 1.035 und 1.020 im Nachauflaufverfahren gegen eine Vielzahl weit verbreiteter breitblättriger Pflanzen sehr starke herbizide Wirkung entfalten.

Die Verbindung 1,076 eignet sich darüberhinaus auch zur Bekämpfung grasartiger Pflanzen, z.B. Hühnerhirse (Tab. C).

In Anbetracht der Vielseitigkeit der Applikationsmethoden können die erfindungsgemäßen Verbindungen bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor - Färberdistel |
| Carya illinoinensis | Pekannußbaum |

| Botanischer Name | Deutscher Name |
|---|---|
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum,Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |

| Botanischer Name | Deutscher Name |
|---|---|
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Triticum durum | Hartweizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die neuen Verbindungen mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Chinolincarbonsäuren und andere in Betracht.

Außerdem kann es von Nutzen sein, die neuen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Tabelle A

Herbizide Wirkungen bei Nachauflaufanwendung von 0,5 kg a.S./ha im Gewächshaus

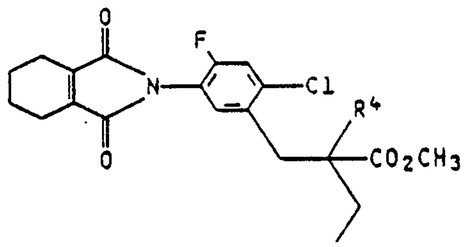

| Bsp. | $R^4$ | Testpflanzen und Schädigung (%) | | | |
|---|---|---|---|---|---|
| | | AMARE | GALAP | IPOSS | CENCY |
| 1.058 | Ethyl | 100 | 98 | 100 | 100 |
| 1.046 | Methyl | 100 | 100 | 100 | 100 |

Tabelle B

Herbizide Wirkungen bei Nachauflaufanwendung von 0,5 kg a.S./ha im Gewächshaus

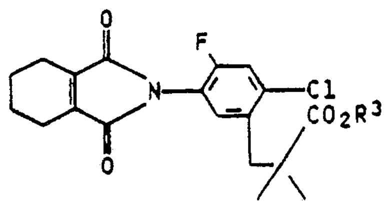

| Bsp. | $R^3$ | Testpflanzen und Schädigung (%) | | |
|---|---|---|---|---|
| | | GALAP | IPOSS | MERAN |
| 1.032 | $CH_2CH_2OCH_3$ | 100 | 100 | 100 |
| 1.035 | $CH_2CH_2OCH_2CH_3$ | 100 | 100 | 100 |

Tabelle C

Herbizide Wirkung von Bsp. 1.076 bei Nachauflaufanwendung von 0,5 kg a.S./ha im Gewächshaus

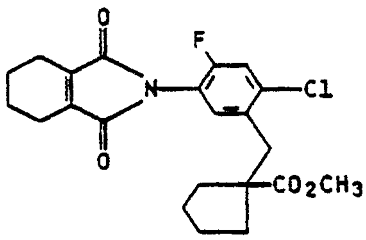

| Testpflanzen | Schädigung |
|---|---|
| ECHCG | 100 |
| AMARE | 100 |
| GALAP | 100 |
| IPOSS | 100 |
| CENCY | 100 |
| CASTO | 100 |

Tabelle D

Herbizide Wirkung von Bsp. 1.020 bei Nachauflaufanwendung von 0,5 kg a.S./ha im Gewächshaus

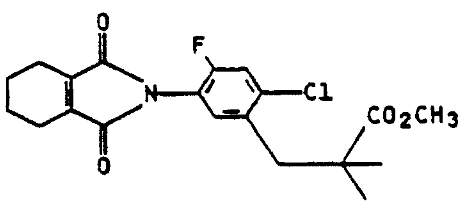

| Testpflanzen | Schädigung |
|---|---|
| IPOSS | 100 |
| CENCY | 100 |
| MERAN | 100 |
| VIOSS | 100 |

## Ansprüche

1. 3-Phenylpropionsäurederivate der allgemeinen Formel I,

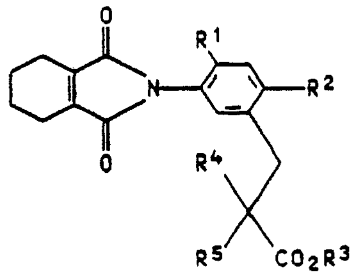

I

in der bedeutet:
$R^1$ Wasserstoff oder Halogen,
$R^2$ Halogen,
$R^3$ unsubstituiertes oder durch Halogen, $C_1$-$C_4$-Alkoxy oder $C_1$-$C_4$-Alkylthio substituiertes $C_2$-$C_6$-Alkyl, unsubstituiertes oder durch $C_5$-$C_7$-Cycloalkyl substituiertes $C_1$-$C_6$-Alkyl, ferner $C_5$-$C_7$-Cycloalkyl, $C_3$-$C_6$-Alkenyl oder $C_3$-$C_6$-Alkinyl oder mit $R^4$ gemeinsam eine $C_2$-$C_4$-Alkylenkette,
$R^4$ Wasserstoff oder unsubstituiertes oder durch Halogen, $C_1$-$C_4$-Alkoxy oder $C_1$-$C_4$-Alkylthio oder $C_5$-$C_7$-Cycloalkyl substituiertes $C_1$-$C_6$-Alkyl; $C_3$-$C_7$-Cycloalkyl; unsubstituiertes oder durch Halogen substituiertes $C_3$-$C_6$-Alkenyl oder $C_3$-$C_6$-Alkinyl oder Halogen oder
$R^4$ gemeinsam mit $R^5$ und dem Kohlenstoffatom dieses Substituenten $C_3$-$C_6$-Cycloalkyl, das durch ein Sauerstoffatom oder ein Schwefelatom unterbrochen sein kann,
$R^5$ unsubstituiertes $C_1$-$C_6$-Alkyl oder durch Halogen, $C_1$-$C_4$-Alkoxy oder $C_1$-$C_4$-Alkylthio substituiertes $C_2$-$C_6$-Alkyl; Cyano oder Acyl ($COR^6$) oder Alkoxycarbonyl ($CO_2R^7$), wobei bedeutet:
$R^6$ $C_1$-$C_6$ Alkyl oder gemeinsam mit $R^4$ eine $C_3$-$C_5$-Alkylenkette und $R^7$ unsubstituiertes oder durch Halogen, $C_1$-$C_4$-Alkoxy oder $C_1$-$C_4$-Alkylthio substituiertes $C_2$-$C_6$-Alkyl oder unsubstituiertes oder durch $C_5$-$C_7$-Cycloalkyl substituiertes $C_1$-$C_6$-Alkyl, ferner $C_5$-$C_7$-Cycloalkyl, $C_3$-$C_6$-Alkenyl oder $C_3$-$C_6$-Alkinyl
mit der Maßgabe, daß $R^1$ und $R^4$ nicht gleichzeitig die Bedeutung Wasserstoff haben können, wenn $R^5$ Cyano oder $CO_2R^7$ ist.

2. Verfahren zur Herstellung von Verbindungen der Formel I gemäß Anspruch 1, worin $R^5$ Cyano, $COR^6$ oder $CO_2R^7$ bedeutet, dadurch gekennzeichnet, daß man ein entsprechend substituiertes Benzylhalogenid der Formel II,

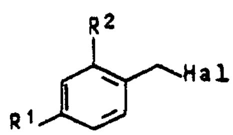

II

worin Hal vorzugsweise Chlor oder Brom bedeutet, in einem Lösungsmittel in Gegenwart einer Base mit einem entsprechenden Ester der Formel III,

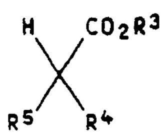

III

in an sich bekannter Weise zu einem Ester der Formel IV umsetzt,

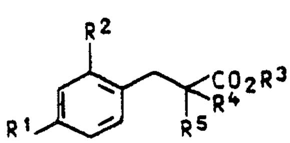

IV

diesen zur Nitroverbindungen V nitriert und reduziert (VI)

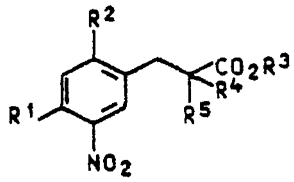

V

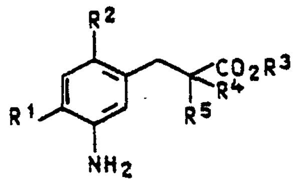

VI

und die Aminoverbindung VI mit Tetrahydrophthalsäureanhydrid umsetzt.

3. Verfahren zur Herstellung von Verbindungen der Formel I gemäß Anspruch 1, worin $R^5$ Cyano, $COR^6$ oder $CO_2R^7$ bedeutet, dadurch gekennzeichnet, daß man einen entsprechend substituierten 3-Phenylpropionsäureester der Formel VII

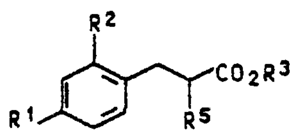

VII

in an sich bekannter Weise in Gegenwart einer Base mit einer Halogenverbindung $R^4$-X, worin X vorzugsweise Chlor, ferner Brom oder Iod oder, sofern $R^4$ die Bedeutung Halogen besitzt Sulfuryl, bedeutet, zum Ester IV umsetzt, zu V nitriert, reduziert (VI) und mit Tetrahydrophthalsäureanhydrid zum Tetrahydrophthalimid der Formel I in an sich bekannter Weise umsetzt.

4. Verfahren zur Herstellung von Verbindungen der Formel I gemäß Anspruch 1, wobei $R^4$ jedoch nicht Wasserstoff oder Halogen und $R^5$ nicht Cyano, $COR^6$ oder $CO_2R^7$ bedeutet, dadurch gekennzeichnet, daß man ein entsprechend substituiertes Benzylhalogenid der Formel II gemäß Anspruch 2 in an sich bekannter Weise in einem aprotischen Lösungsmittel in Gegenwart einer Base und gegebenenfalls eines Katalysators mit einem Aldehyd der Formel IX, zum Propionaldehyd der Formel X umsetzt,

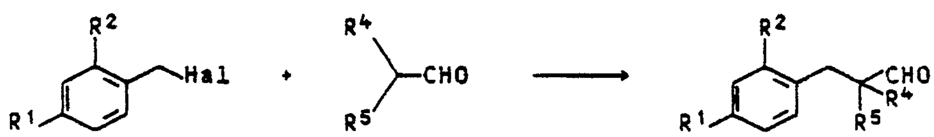

II IX X

diesen in an sich bekannter Weise zur Carbonsäure der Formel

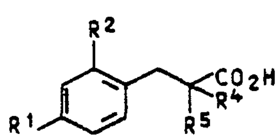

XI

oxidiert, verestert und nach Anspruch 2 oder 3 zu einer Verbindung der Formel I gemäß Anspruch 1 umsetzt.

5. Verwendung von Verbindungen der Formel I gemäß Anspruch 1 als Herbizide.

6. Herbizid wirkendes Mittel, enthaltend eine Verbindung der Formel I gemäß Anspruch 1 und übliche Hilfsstoffe, Streck- und Verdünnungsmittel.